# EUROPÄISCHE PATENTSCHRIFT

(11) **EP 1 311 590 B1**
(45) Veröffentlichungstag und Bekanntmachung des Hinweises auf die Patenterteilung: **22.03.2006**
(21) Anmeldenummer: 01962879.1
(22) Anmeldetag: 27.07.2001
(51) Int. Cl.: C08G 77/54, C08G 77/46, D06M 15/643, D06M 15/647, A61K 8/00, A61Q 5/00

(54) **POLYSILOXANPOLYMERE, VERFAHREN ZU IHRER HERSTELLUNG UND IHRE VERWENDUNG**
POLYSILOXANE POLYMERS, METHOD FOR THEIR PRODUCTION AND THE USE THEREOF
POLYMERES POLYSILOXANES, PROCEDE DE PRODUCTION ET UTILISATION

(30) Priorität: 27.07.2000 DE 10036536
(43) Veröffentlichungstag der Anmeldung: 21.05.2003
(73) Patentinhaber: GE Bayer Silicones GmbH & Co. KG, 40699 Erkrath (DE)
(72) Erfinder: LANGE, Horst, 44879 Bochum (DE); WAGNER, Roland, 51061 Köln (DE); WITOSSEK, Anita, 40764 Langenfeld (DE); STACHULLA, Karl-Heinz, 51379 Leverkusen (DE); TEUBER, Siegfried, 47799 Krefeld (DE); SCHNERING, Albert, 51067 Köln (DE); MÖLLER, Annette, 51381 Leverkusen (DE); KROPFGANS, Martin, 51519 Odenthal (DE); SOCKEL, Karl-Heinz, 51373 Leverkusen (DE); FIRSTENBERG, Don, 40699 Erkrath (DE)
(86) Internationale Anmeldenummer: PCT/EP2001/008695
(87) Internationale Veröffentlichungsnummer: WO 2002/010259

(56) Entgegenhaltungen:
- EP-A- 0 282 720
- EP-A- 1 000 959
- WO-A-01/41720
- WO-A-99/50338
- DE-A- 19 817 776
- FR-A- 2 535 730

## Beschreibung

Die Erfindung betrifft Polysiloxanpolymere vorzugsweise zur Verwendung als waschbeständige hydrophile Weichmacher auf Basis quartärer Ammoniumgruppen enthaltender Siloxane, Verfahren zu deren Herstellung und deren Verwendung vorzugsweise als Weichmacher.

Aminogruppen enthaltende Polysiloxane sind als textile Weichmacher aus der EP-A-0 441 530 bekannt. Die Einführung von durch Ethylenoxid-/Propylenoxideinheiten modifizierten Aminostrukturen als Seitenketten bewirkt eine Verbesserung des Effekts wie in der US 5 591 880 und US 5 650 529 beschrieben. Die Alkylenoxideinheiten erlauben hierbei die gezielte Einstellung der hydrophilen-hydrophoben Balance. Nachteilig ist für den Aufbau der Verbindungen ist die bei der Synthese eingeschlossene schwierige Veresterung von Aminoalkoholen mit siloxangebundenen Carbonsäuregruppen. Zusätzlich liegt eine Schwierigkeit in den weichmachenden Eigenschaften , die durch die generelle Kammstruktur der Produkte induziert werden. Zur Beseitigung dieser Nachteile ist gemäß der US 5 807 956 und US 5 981 681 vorgeschlagen worden, α,ω-epoxymodifizierte Siloxane mit α,ω-aminofunktionalisierten Alkylenoxiden umzusetzen, und diese Produkte als hydrophile Weichmacher einzusetzen.

Zur Verbesserung der Substantivität sind Versuche unternommen worden, quartäre Ammoniumgruppen in alkylenoxidmodifizierte Siloxane einzuführen. Die US 5 625 024 offenbart verzweigte alkylenoxidmodifizierte polyquartäre Polysiloxane, die aus α,ω-OH terminierten Polysiloxanen und Trialkoxysilanen durch Kondensation synthetisiert worden sind. Die quartäre Ammoniumstruktur wird über das Silan eingebracht, wobei das quartäre Stickstoffatom durch Alkylenoxideinheiten substituiert ist. Streng kammartige alkylenoxidmodifizierte polyquartäre Polysiloxane sind ebenfalls in der US 5 098 979 beschrieben worden. Die Hydroxylgruppen von kammartig substituierten Polyethersiloxanen werden mit Epichlorhydrin in die entsprechenden Chlorhydrinderivate überführt. Anschließend erfolgt eine Quatemierung mit tertiären Aminen. Nachteilig an dieser Strategie ist der notwendige Umgang mit Epichlorhydrin und die relativ geringe Reaktivität der Chlorhydrin-Gruppierung während der Quaternierung.

Aus diesem Grund heraus sind die Hydroxylgruppen kammartig substituierter Polyethersiloxane alternativ mit Chloressigsäure verestert worden. Durch die Carbonylaktivierung kann die abschließende Quaternierung erleichtert vollzogen werden wie in der US 5 153 294 und US 5 166 297 bereits beschrieben.

Die nach dem Prioritätstag dieser Anmeldung veröffentlichten WO 01/41719 und WO 01/41720 beschreiben quartäre Polysiloxanverbindungen zur Verwendung in kosmetischen Zusammensetzungen.

Die Reaktion von α,ω-Diepoxiden mit tertiären Aminen in Gegenwart von Säuren liefert α,ω-diquartäre Siloxane, welche zu Haarpflegezwecken eingesetzt werden können, ist bereits in der DE-PS 37 19 086 offenbart. Eine Verringerung der Auswaschbarkeit von Pflegesubstanzen aus den Haaren kann erzielt werden, wenn die α,ω-Diepoxide mit di-tertiären Aminen in Gegenwart von Säuren zu langkettigen polyquartären Polysiloxanen umgesetzt werden, wie in der EP-A-0 282 720 offenbart. Allerdings bezieht sich die Auswaschbeständigkeit aus Haaren auf den kurzzeitigen Angriff von vornehmlich Wasser und sehr milden, die Haut nicht irritierenden Tensiden, während waschbeständige, hydrophile Weichmacher für Textilien dem Angriff konzentrierter Tensidlösungen mit hohem Fett- und Schmutzlösevermögen zu widerstehen haben. Erschwerend kommt hinzu, daß moderne Waschmittel stark alkalische Komplexbildner, oxydativ wirkende Bleichmittel und komplexe Enzymsysteme enthalten und die Fasern der Einwirkung oftmals über Stunden bei erhöhten Temperaturen ausgesetzt sind.

Aus diesem Grund wird ein grundsätzlich anderer Ansatz in DE-OS 32 36 466 beschrieben. Die Umsetzung von OH-terminierten Siloxanen mit quartären Ammoniumstrukturen enthaltenden Alkoxysilanen liefert reaktive Zwischenprodukte, die mit geeigneten Vernetzungsagenzien, wie Trialkoxysilanen, auf der Faseroberfläche zu waschbeständigen Schichten vernetzen sollen. Wesentlicher Nachteil dieses Ansatzes ist, daß die über Stunden notwendige Stabilität eines wässrigen Ausrüstungsbades nicht garantiert werden kann und unvorhergesehene Vernetzungsreaktionen im Bad bereits vor der Textilausrüstung auftreten können.

Die beschriebenen Vorschläge stellen keine befriedigenden Lösungen für das Problem dar, den für forfschrittliche Silicone typischen weichen Griff und die ausgeprägte Hydrophilie nach Erstausrüstung eines Textilmaterials auch dann zu erhalten, wenn dieses dem Angriff aggressiver Detergenzienformulierungen im Verlauf wiederholter Waschprozesse bei gegebenenfalls erhöhter Temperatur ausgesetzt wird.

Es ist somit Aufgabe der Erfindung gewesen, Polysiloxanverbindungen, insbesondere polyquartäre Polysilicone, deren Herstellung und deren Verwendung als waschbeständige hydrophile Weichmacher zur Verfügung zu stellen, die nicht die Nachteile des Standes der Technik aufweisen.

Die erfindungsgemäßen Polysiloxanverbindungen, insbesondere die polyquartären Silicone sollen auf den Textilien nach entsprechender Applikation einen silicontypischen weichen Griff und eine ausgeprägte Hydrophilie verleihen und dieses Eigenschaftsbild auch nach Einwirkung von Detergenzienformulierungen während wiederholter Waschprozesse bei gegebenenfalls erhöhter Temperatur nicht verloren geht.

Diese Aufgabe wird gelöst durch Polysiloxanpolymere mit den Wiederholungseinheiten 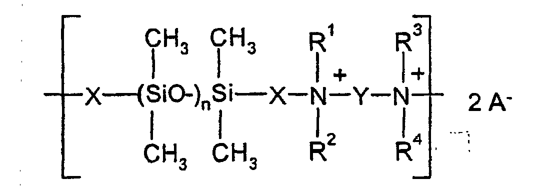 und 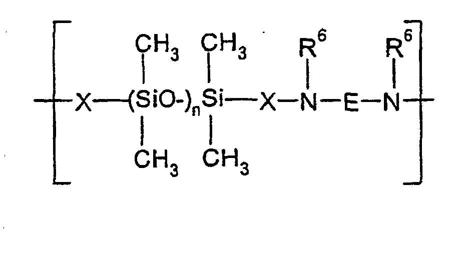 worin
- X: ein zweiwertiger Kohlenwasserstoffrest mit mindestens 4 Kohlenstoffatomen ist, der eine Hydroxylgruppe aufweist und der durch ein Sauerstoffatom unterbrochen sein kann, und die Gruppen X in den Wiederholungseinheiten gleich oder verschieden sein können,
- Y: ein zweiwertiger Kohlenwasserstoffrest mit mindestens 2 Kohlenstoffatomen ist, der eine Hydroxylgruppe aufweisen und der durch ein oder mehrere Sauerstoff- oder Stickstoffatome, bevorzugt ein Sauerstoffatom oder ein Stickstoffatom unterbrochen sein kann,
- R¹, R², R³ und R⁴: gleich oder verschieden sind und Alkylreste mit 1 bis 4 Kohlenstoffatomen oder Benzylreste bedeuten oder jeweils die Reste R¹ und R³ oder R² und R⁴ Bestandteile eines verbrückenden Alkylenrestes sein können,
- R⁶: H oder ein Alkylrest mit 1 bis 20 Kohlenstoffatomen ist, der sauerstoffsubstituiert sein kann,
- E: die Struktur -B-O-(EOx)ᵥ(POx)_{w}-B-, worin EOx eine Ethylenoxideinheit und POx eine Propylenoxideinheit ist, und
- B: gradkettiges oder verzweigtes C₂ bis C₆ Alkylen,
- v: 0 bis 200,
- w: 0 bis 200,
- v+w: ≥ 1 entspricht,
- n: 2 bis 1000, worin die n in den Wiederholungseinheiten gleich oder verschieden sein können,
- A⁻: ein anorganisches oder organisches Anion bedeutet.

Bei den erfindungsgemäßen Polysiloxanverbindungen handelt es sich um lineare oder cyclische Polysiloxanpolymere.

Wenn es sich um lineare Polysiloxanpolymere handelt, werden die terminalen Gruppen zweckmäßig ausgewählt aus 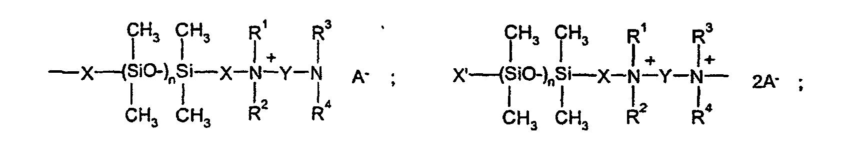 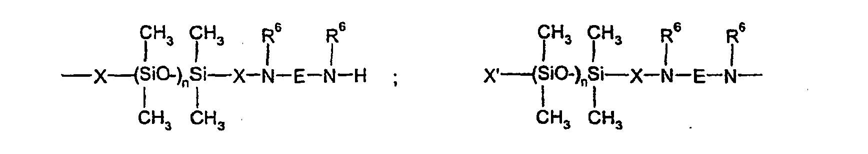 und/oder 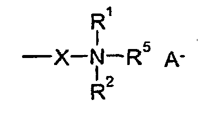 worin X, R¹, R², R³, R⁴, R⁶, Y, E, A⁻ und n wie oben definiert sind,
X' ein Kohlenwasserstoffrest mit mindestens 4 Kohlenstoffatomen ist, der eine Epoxygruppe oder eine mit Alkoholen, Wasser oder Aminen geöffnete Epoxygruppe aufweist und der durch ein Sauerstoffatom unterbrochen sein kann, und
R⁵ ein Alkylrest mit 1 bis 20 Kohlenstoffatomen ist, und
wobei die endständigen Gruppen X in den terminalen Gruppen jeweils an die endständigen Stickstoffatome der Wiederholungseinheiten und die endständigen

Stickstoffatome in den terminalen Gruppen jeweils an die endständigen Gruppen X der Wiederholungseinheiten binden.

Die vier ersten oben genannten tenninalen Gruppen resultieren dabei aus den bei der Herstellung verwendeten, unten beschriebenen Bisaminen, während die letzte terminale Gruppe aus einem ggf. bei der Herstellung zugesetzten Monoamin resultiert.

Die oben in der Definition von X' erwähnte Epoxygruppe ist bevorzugt eine endständige Epoxygruppe. Besonders bevorzugt wird die Gruppe X' ausgewählt aus folgenden Formeln: 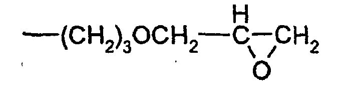 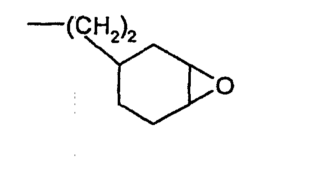 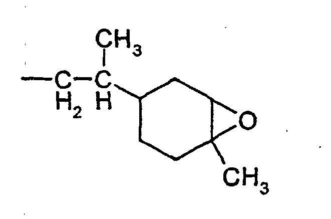 sowie durch Alkohole, Wasser oder Amine geöffnete Epoxidstrukturen davon. Bei der Öffnung mit Alkoholen resultieren durch eine Hydroxylgruppe und eine Ethergruppe substituierte Reste, bei der Öffnung mit Wasser resultieren mit zwei Hydroxylgruppen substituierte Reste und bei der Öffnung mit Aminen resultieren Hydroxylamin-Reste. Dies ist dem Fachmann an sich bekannt.

Bei einer bevorzugten Ausführungsform der erfindungsgemäßen Polysiloxanpolymere handelt es sich cyclische polyquarternäre Polysiloxanpolymere der allgemeinen Formel (I) 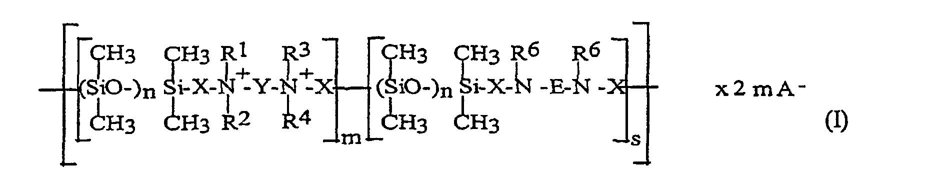 und/oder linearen Verbindungen der allgemeinen Formel (Il) 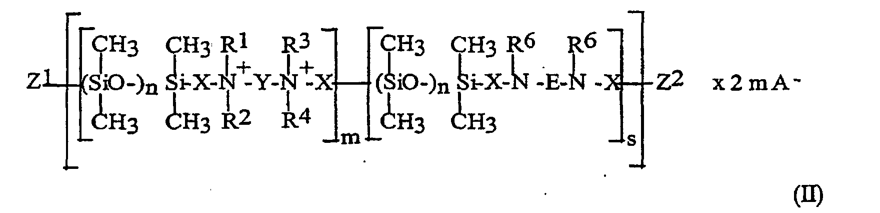 worin
- X: ein zweiwertiger Kohlenwasserstoffrest mit mindestens 4 Kohlenstoffatomen ist, der eine Hydroxylgruppe aufweist und der durch ein Sauerstoffatom unterbrochen sein kann,
- Y: ein zweiwertiger Kohlenwasserstoffrest mit mindestens 2 Kohlenstoffatomen ist, der eine Hydroxylgruppe aufweisen und der durch ein oder mehrere Sauerstoff- oder Stickstoffatome unterbrochen sein kann,
- Z¹: ein H, OH, ein Alkyl- Epoxy- oder ein Alkoxyrest ist, oder die Bedeutung eines Kohlenwasserstoffrestes mit mindestens 4 Kohlenstoffatomen hat, der eine oder mehrere Hydroxylgruppe(n) aufweist und durch eine oder mehrere Sauerstoffatome unterbrochen sein kann oder die Bedeutung des Restes 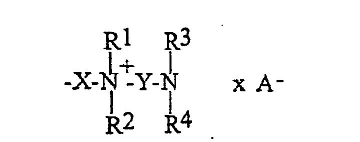
oder 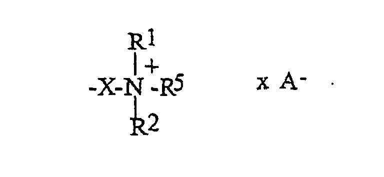 hat, wobei R⁵ ein Alkylrest mit 1 bis 20 Kohlenstoffatomen ist,
Z² die Gruppe 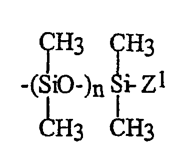
- R¹, R², R³ und R⁴: gleich oder verschieden sind und Alkylreste mit 1 bis 4 Kohlenstoffatomen oder Benzylreste bedeuten oder jeweils die Reste R¹ und R³ oder R² und R⁴ Bestandteile eines verbrückenden Alkylenrestes sein können,
- R⁶: H oder ein Alkylrest mit 1 bis 20 Kohlenstoffatomen, der sauerstoffsubstituiert sein kann,
- E: die Struktur -B-(OCH₂CH₂)ᵥ(OCH₂CH(CH₃))_{w}-O-B- mit
- B: gradkettig oder verzweigt C₂ bis C₆ Alkylen,
- v: 0 bis 200,
- w: 0 bis 200,
- v+w: ≥ 1 entspricht,
- A⁻: ein anorganisches oder organisches Anion
- n: 5 bis 200
- m: ganze Zahl ≥ 1 und
- s: ganze Zahl ≥ 1
bedeuten.

In einer besonderen Ausführungsform der Polysiloxanpolymere der Erfindung ist n = 5 bis 82.

In einer weiteren besonderen Ausführungsform der Polysiloxanpolymere der Erfindung ist n = 5 bis 20.

Die erfindungsgemäßen Polysiloxan-Verbindungen insbesondere der Formeln (I) und (II) können als waschbeständige hydrophile Weichmacher für die textile Erstausrüstung und als Weichmacher in auf nichtionogenen oder anionischen/nichtionogenen Tensiden beruhenden Formulierungen zur Wäsche von Fasern und Textilen verwendet werden.

In einer bevorzugten Ausführungsform der vorliegenden Erfindung steht X für einen Rest aus der Gruppe 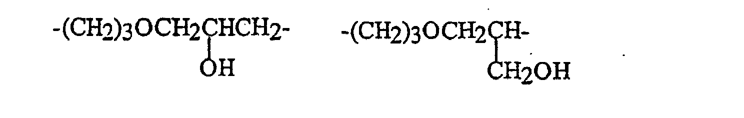 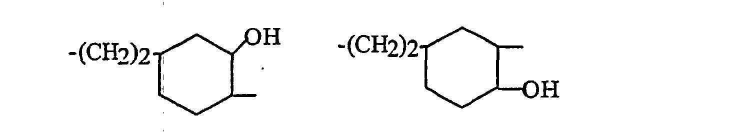 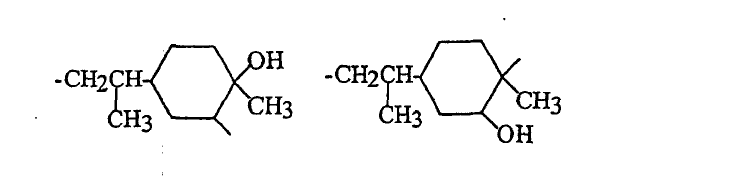 wobei die Anbindung an die Polysiloxaneinheit von links erfolgt.

In einer weiteren Ausführungsform der vorliegenden Erfindung steht Y für einen Rest -(CH₂)ₒ-, mit o von 2 bis 6.

In einer weiteren bevorzugten Ausführungsform der vorliegenden Erfindung bedeuten die Reste R¹, R², R³, R⁴ bevorzugt Methylreste.

In einer weiteren bevorzugten Ausführungsform der vorliegenden Erfindung bedeutet R⁶ -CH₂CH₂OH, -CH₃ oder Wasserstoff. Besonders bevorzugt ist R⁶ Wasserstoff.

Die Gruppe E in den erfindungsgemäßen Polysiloxanverbindungen wird durch die Struktur -B-O-(EOx)ᵥ(POx)_{w}-B- dargestellt, worin EOx für eine Ethylenoxideinheit steht und POx für eine Propylenoxideinheit steht. Dabei kann die Gruppe -(EOx)ᵥ(POx)_{w}- für Polyethylenoxidpolymergruppen, Polypropylenoxidpolymergruppen und Polyethylenoxid-Polypropylenoxid-Copolymergruppen stehen. Bei den Polyethylenoxid-Polypropylenoxid-Copolymergruppen kann es sich statistische oder blockartig aufgebaute Copolymergruppen handeln. Besonders bevorzugt sind Polyethylenoxid-Polypropylenoxid-Blockcopolymergruppen mit beliebiger Anordnung von mindestens einer Polyethylenoxidgruppe und mindestens einer Polypropylenoxidgruppe. Letztere sind insbesondere Bestandteile der im Handel unter der Bezeichnung Jeffamine erhältlichen Diamine.

In einer weiteren bevorzugten Ausführungsform der vorliegenden Erfindung steht B für -CH₂CH₂- und -CH₂CH(CH₃)- Einheiten.

In einer weiteren bevorzugten Ausführungsform der vorliegenden Erfindung steht v für einen Bereich von 0 bis 100, bevorzugt 0 bis 70, und besonders bevorzugt 0 bis 40.

In einer weiteren bevorzugten Ausführungsform der vorliegenden Erfindung steht w für einen Bereich von 0 bis 100, bevorzugt 0 bis 70, besonders bevorzugt 0 bis 40.

In einer weiteren Ausführungsform der vorliegenden Erfindung ist das Verhältnis m/(m+s) x100 in den allgemeinen Formeln (I) und (II) 0,1 bis 99,9 %. Das Verhältnis m/(m+s) x 100 stellt den Prozentanteil der Anzahl Wiederholungseinheiten der Formel 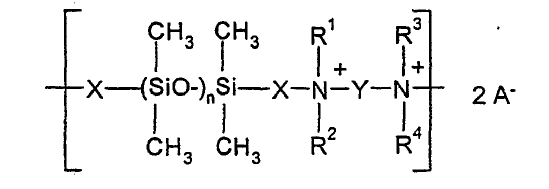 zur Gesamtzahl der Wiederholungseinheiten von 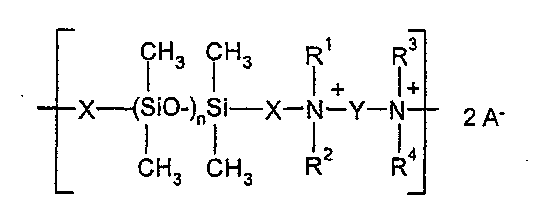 und 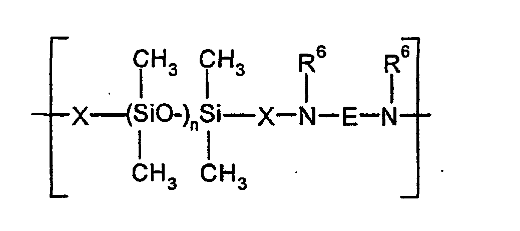 im erfindungsgemäßen Polysiloxanpolymer dar.

Mit anderen Worten ist im erfindungsgemäßen Polysiloxanpolymer das Verhältnis der Anzahl der Wiederholungseinheiten der Formel 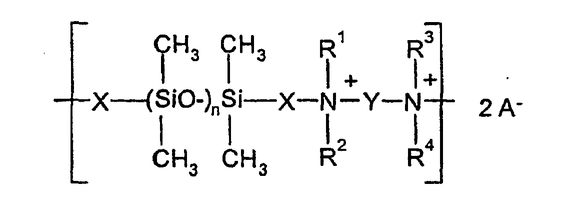 zur Anzahl der Wiederholungseinheiten der Formel 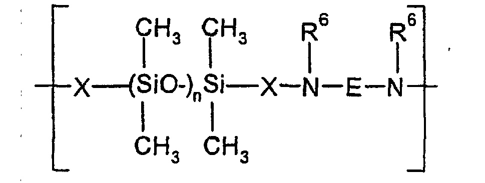 zweckmäßig etwa 1 : 1000 bis etwa 1000 : 1, bevorzugt 1 : 1 bis 100 : 1, besonders bevorzugt 4 : 1 bis 20 : 1, und ganz besonders bevorzugt 3 : 1 bis 10 : 1, am meisten bevorzugt 3 : 1 bis 9 : 1.

Durch die geeignete Auswahl des obigen Verhältnisses der Wiederholungseinheiten lassen sich die Eigenschaften der erfindungsgemäßen Polysiloxane, wie zum Beispiel die Substantivität (Aufziehverhalten) bezüglich der zu behandelnden Substrate, wie Cellulosefasem, Haare, Polyamidfasern, polare Lack- oder Kunststoffoberflächen etc. steuern und daran anpassen. Weiterhin kann das Auswaschverhalten, die Löslichkeit in Waschmittelflotten bzw. Tensidlösungen teilweise über das vorstehend erwähnte Verhältnis gesteuert und an Waschmittelformulierungen angepasst werden.

Die Beeinflussung weiterer Eigenschaften der erfindungsgemäßen Polysiloxane ist weiterhin über die Steuerung des Molekulargewichtes möglich, welches wiederum beispielsweise durch den Zusatz von Monoaminen der Formel 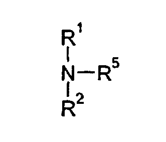 worin R¹, R² und R⁵ wie oben definiert sind, wobei die genannten Definitionen von denen der übrigen Gruppen R¹, R² im Molekül gleich oder verschieden sein können, während der Polymerisation in an sich bekannter Weise gesteuert werden kann. Bevorzugte Monoamine sind beispielsweise Trimethylamin, Triethylamin, Tripropylamin, Tributylamin oder Benzyldimethylamin.

In einer bevorzugten Ausführungsform werden Monoamine als Kettenabrecher berwendet. Werden Monoamine der o.g. Formel als Kettenabbrecher verwendet, so beträgt ihr Anteil beispielsweise molar maximal 20%, 10%, 5%, und oder 1 % des Gehaltes an difunktionellen Aminwiederholungseinheiten der o.g. Formeln, woraus sich beispielsweise ein entsprechender Bereich von 1 bis 20% ergibt.

Vorzugsweise stehen für das Anion A' physiologisch vertretbare anorganische Reste, wie Chlorid, Bromid, Hydrogensulfat, Sulfat, etc. bzw. organische Reste aus der Gruppe bestehend aus Acetat, Propionat, Octanoat, Decanoat, Dodecanoat, Tetradecanoat, Hexadecanoat, Octadecanoat, Oleat.

Ein weiterer Gegenstand der vorliegenden Erfindung ist ein Verfahren zur Herstellung der erfindungsgemäßen Polysiloxan-Verbindungen dadurch gekennzeichnet, dass man Bisepoxid-terminierte Polysiloxane der Formel 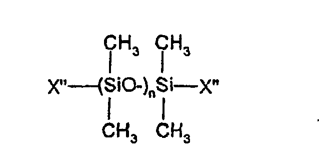 worin X" ein zweiwertiger Kohlenwasserstoffrest mit mindestens 4 Kohlenstoffatomen ist, der eine Epoxygruppe aufweist und der durch ein Sauerstoffatom unterbrochen sein kann, mit Bisaminen der Formeln 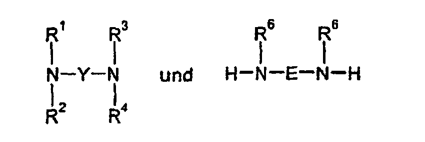 in geeigneter Reihenfolge, gegebenenfalls unter Zusatz eines Monoamins der Formel 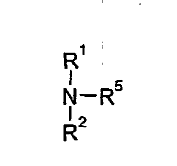 umsetzt, worin die Substituenten wie oben definiert sind. Die Erfindung betrifft auch die nach dem vorstehend beschriebenen Verfahren erhältlichen Polysiloxanverbindungen.

In einer bevorzugten Ausführungsform des erfindungsgemäßen Verfahrens zur Herstellung der erfindungsgemäßen Polysiloxanverbindungen, insbesondere der allgemeinen Formel (I) und (II) sind der Ausgangspunkt für die Synthese α,ω-Si-H funktionalisierte Siloxane der allgemeinen Struktur 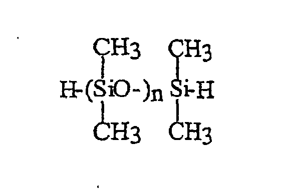 worin n wie oben definiert ist.

Sofern nicht kommerziell erhältlich, können diese Siloxane nach bekannten Verfahren, z.B. durch Äquilbrierung hergestellt werden (Silicone, Chemie und Technologie, Vulkan-Verlag, Essen 1989, S. 82-84).

Durch Hydrosilylierung werden zunächst reaktive epoxymodifizierte Zwischenprodukte erzeugt, welche in einem nachfolgenden Schritt alkyliert werden können. Geeignete Ausgangsstoffe zur Erzeugung reaktiver Zwischenstufen sind epoxyfunktionelle Alkene, beispielsweise Vinylcyclohexenoxid und Allylglycidether. Die allgemeine Durchführung von Hydrosilylierungen mit Vertretern der genannten Stoffgruppe ist ebenfalls bekannt (B. Marciniec, Comprehensive Handbook on Hydrosilylation, Pergamon Press, Oxford 1992, S.127-130).

In einem nachfolgenden Schritt werden die epoxymodifizierten Zwischenstufen dann mit di-tertiären Aminen und vorzugsweise zwei primäre oder sekundäre Aminofunktionen tragenden Alkylenoxidderivaten zur Reaktion gebracht Entscheidend ist hierbei, das eine Gesamtstöchiometrie von Epoxygruppen zur Σ(primäre+ sekundäre+tertiäre) der Aminogruppen, 1 : 1 eingehalten wird. Bevorzugt wird auf den Anteil an tertiären Aminogruppen äquimolar Säure HA eingesetzt.

Aminomodifizierte Alkylenoxidderivate besitzen zweckmäßig die Struktur 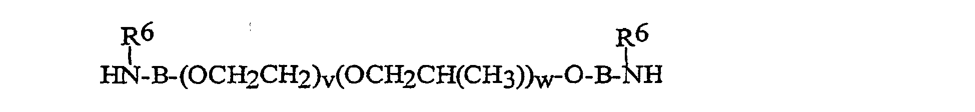 Bevorzugt sind dabei Ethylenoxid-Propylenoxid-Blockcopolymere.

Derartige Amine sind kommerziell unter der Bezeichnung Jeffamine® (Huntsman Corp.) erhältlich. Ein bevorzugtes Beispiel stellt
H₂NCH (CH₃)CH₂[OCH(CH₃)CH₂]ₐ(OCH₂CH₂)_{c}[OCH₂CH(CH₃)]_{b}NH₂ dar, mit a+b = 2- 20 c= 1- 100.

D.h. die erfindungsgemäßen Polysiloxanverbindungen sind zweckmäßig erhältlich durch Umsetzung von Bisepoxid-terminierten Polysiloxanverbindungen mit Diaminen der Formel 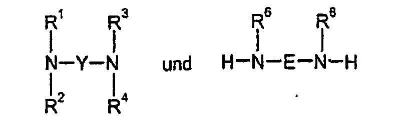 vorzugsweise in Gegenwart von Säuren und gegebenenfalls in Gegenwart von Monoaminen der Formel

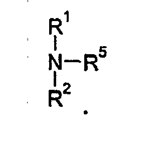

In einem bevorzugten Verfahren zur Herstellung der erfindungsgemäßen Polysiloxanverbindungen insbesondere der Formeln (I) und (II) werden α,ω Si-H funktionalisierte Siloxane der allgemeinen Struktur 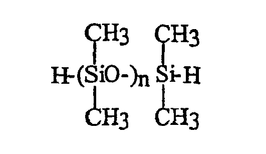 mit, bezogen auf SiH-Gruppen 1,0 bis 1,5 mol eines Epoxides, mit endständigen olefinischen Bindungen, worin das Epoxid mindestens 4 Kohlenstoffatome besitzt und zusätzlich eine nichtcyclische Ethergruppe enthalten kann, in Gegenwart eines Hydrosilylierungskatalysators bei Temperaturen von 50 bis 150 °C umsetzt, der Überschuß an olefinischem Epoxid ggf. entfernt, und das Reaktionsprodukt mit einer Mischung aus einem di-tertiären Amin der Formel 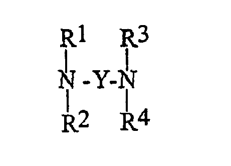 und einem alkylenoxidmodifizierten Diamin der Struktur 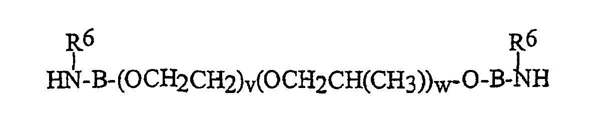 in Gegenwart von Säuren HA bei 40 bis 120°C umgesetzt wird, worin das molare Verhältnis der Epoxygruppen zu tertiären Aminogruppen zu Säuren HA ein Verhältnis von 1 : 1 : 1 und die Gesamtstöchiometrie von Epoxygruppen zu der Summe der (primäre+ sekundäre+tertiäre) Aminogruppen ebenfalls 1 : 1 beträgt. Die Erfindung betrifft auch die nach dem vorstehend beschriebenen Verfahren erhältlichen Polysiloxanverbindungen.

In einer bevorzugten Ausführungsform des Verfahrens zur Herstellung der erfindungsgemäßen Polysiloxanverbindungen insbesondere der Formeln (I) und (II) beträgt das molare Verhältnis der Epoxygruppen zu der Summe (primäre + sekundäre + tertiäre) der Aminogruppen und zur Säuren HA 1 : 1 : 1.

In einer bevorzugten Ausführungsform des Verfahrens zur Herstellung der erfindungsgemäßen Polysiloxanverbindungen insbesondere der Formeln (I) und (II) werden die verschiedenen Aminogruppen tragenden Spezies und eine äquimolare Menge an Säure HA gemeinsam dem Ansatz hinzugefügt werden.

Obwohl die Verwendung entsprechender Diaminoderivate bevorzugt ist, kann auch partiell auf analoge, trifunktionelle oder monofunktionelle Strukturen zurückgegriffen werden, wenn eine anteilige Vernetzung oder aber Kettenterminierung beabsichtigt ist. Der Anteil an trifunktionellen, vernetzenden bzw. monofunktionellen, kettenabbrechenden Aminoderivaten beträgt molar maximal 10%, bevorzugt 5%, und besonders bevorzugt 1 % des Gehaltes an difunktionellem Derivat.

Es liegt ebenfalls im Rahmen der Erfindung, das di-tertiäre Amin anteilig durch monofunktionelle tertiäre Amine zu ersetzen. Deren Anteil beträgt molar ebenfalls maximal 10 %, bevorzugt 5 %, und besonders bevorzugt 1 % des Gehaltes an di-tertiärem Amin.

Hinsichtlich der Reaktionsführung liegt es im Rahmen der Erfindung, die Säure HA über das für die Quartärnierung der tertiären Aminogruppen hinausgehende Maß bis zur molaren Äquivalenz mit allen Aminogruppen hinzuzufügen. Dies bedeutet, daß die erfindungsgemäßen Verbindungen bezüglich der Struktur der Aminogruppen als freie Amine oder aber Aminsalze vorliegen können, so dass beispielsweise im Falle der vollständigen Protonierung (zwei zusätzliche Äquivalente Säure) die Polysiloxanverbindungen Wiederholungseinheiten der folgenden Formel aufweisen:

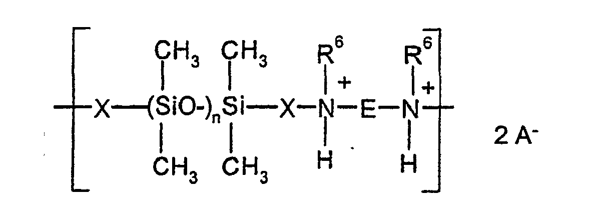

Die verschiedenen Aminogruppen tragenden Spezies könnnen in einer bevorzugten Ausführungsvariante, gegebenenfalls unter Hinzufügung äquimolarer Mengen an Säure HA, gemeinsam dem Ansatz hinzugefügt werden. Es liegt aber auch im Rahmen der Erfindung, zunächst die Epoxyderivate mit den tertiären Aminen in Gegenwart einer zu den tertiären Aminogruppen äquivalenten Menge an Säure HA zur Reaktion zu bringen und nachfolgend die primäre bzw. sekundäre Aminogruppen aufweisenden Alkylenoxidderivate, gegebenenfalls unter Zusatz von Säure HA bis zur Äquivalenz mit den Aminogruppen, hinzuzufügen. Schließlich ist es auch möglich, zunächst die primäre oder sekundäre Aminogruppen tragenden Alkylenoxidderivate, gegebenenfalls in Gegenwart äquimolarer Mengen an Säure HA, mit den Epoxyderivaten reagieren zu lassen und anschließend die Quartänzierung auszuführen.

Es ist weiterhin erfindungsgemäß, mehrere Siloxankomponenten und/oder Alkylenoxidderivate unterschiedlicher jeweiliger Kettenlänge unter Beibehaltung der gewünschten Gesamtstöchiometrie zur Reaktion zu bringen. Es folgt hieraus z.B. die Möglichkeit, eine gewünschte Siloxankettenlänge durch Einsatz einer einzigen Siloxankomponente oder aber durch gezielte Mischung mehrerer Siloxankomponenten einzustellen. Analog dazu ist es möglich, eine vorteilhafte durchschnittliche Alkylenoxidblocklänge in Form einer monomodalen, bimodalen oder polymodalen Verteilung darzustellen.

Die Quartämierungs- und Alkylierungsreaktionen werden bevorzugt in polaren organischen Lösungsmitteln ausgeführt. Geeignet sind z.B. Alkohole aus der Gruppe von Methanol, Ethanol, i-Propanol und n-Butanol; Glycole aus der Gruppe von Ethylenglycol, Diethylenglycol, Triethylenglycol, die Methyl-, Ethyl- und Butylether der genannten Glycole, 1,2-Propylenglycol und 1,3-Propylenglycol, Ketone, wie zum Beispiel Aceton und Methylethylketon; Ester aus der Gruppe von Ethylacetat, Butylacetat und 2-Ethyl-hexylacetat, Ether, wie zum Beispiel Tetrahydrofuran und Nitroverbindungen, wie Nitromethan. Die Wahl des Lösungsmittels richtet sich im Wesentlichen nach der Löslichkeit der Reaktionspartner und der angestrebten Reaktionstemperatur. Die Reaktionen werden vorzugsweise im Bereich von 20 °C bis 150 °C, und bevorzugt im Bereich von 40 °C bis 100 °C ausgeführt.

In EP-A-0 282 720 wird die Verwendung von polyquartären Polysiloxanen in kosmetischen Formulierungen, speziell zur Behandlung von Haaren behandelt. Als Vorteile werden generell eine verbesserte Kämmbarkeit, ein guter Glanz, eine hohe antistatische Effektivität und eine verbesserte Auswaschbeständigkeit genannt.

Die letztgenannte Eigenschaft ist nicht mit einer Waschbeständigkeit in erfindungsgemäßen Sinne gleichzusetzen. Während sich die Auswaschbeständigkeit aus Haaren auf den kurzzeitigen Angriff von vornehmlich Wasser und sehr milden, die Haut nicht irritierenden Tensiden bezieht, haben waschbeständige, hydrophile Weichmacher für Textilien dem Angriff konzentrierter Tensidlösungen mit hohem Fett- und Schmutzlösevermögen zu widerstehen. Diesen Tensidsystemen sind in modernen Waschmitteln stark alkalische Komplexbildner, oxydativ wirkende Bleichmittel und komplexe Enzymsysteme hinzugefügt. Die Einwirkung erfolgt häufig über Stunden bei erhöhten Temperaturen. Aus diesen Gründen heraus ist eine Übertragung von Erfahrungen aus dem Kosmetikbereich auf das Gebiet der waschbeständigen Textilweichmacher nicht möglich. Die im Stand der Technik zitierte DE-OS 32 36 466 zeigt auf, daß zur Erzielung einer waschbeständigen Textilausrüstung auf vemetzungsfähige Systeme zu orientieren gewesen wäre.

Analog dazu war nicht zu erwarten, daß die erfindungsgemäßen Verbindungen als Weichmacher in auf nichtionischen oder anionischen/nichtionischen Tensiden beruhenden Formulierungen zur Wäsche von Fasern und Textilien wirksam sein könnten. Auch in diesen Fällen erfolgt über lange Zeiträume bei erhöhten Temperaturen die Einwirkung der aggressiven Detergenzienformulierung. Erschwerend kommt hinzu, daß die vorgelagerte Modifizierung der Faseroberfläche mit weichmachenden Substanzen entfällt.

Die Erfindung betrifft des weiteren die Verwendung der vorstehend beschriebenen Polysiloxanverbindungen in kosmetischen Formulierungen für die Haut- und Haarpflege, in Polituren für die Behandlung und Ausrüstung harter Oberflächen, in Formulierungen zum Trocknen von Automobilen und anderen harten Oberflächen, zum Beispiel nach maschinellen Wäschen, zur Ausrüstung von Textilien und Textilfasern, als separate Weichmacher nach dem Waschen von Textilien mit nichtionogenen oder anionischen/nichtionogen Detergenzienformulierungen, als Weichmacher in auf nichtionischen oder anionischen/nichtionischen Tensiden beruhenden Formulierungen zur Textilwäsche, sowie als Mittel zur Verhinderung bzw. Rückgängigmachung von Textilverknitterungen.

Die Erfindung betrifft des weiteren die Verwendung der vorstehend beschriebenen Polysiloxanverbindungen als waschbeständige hydrophile Weichmacher für die textile Erstausrüstung.

Ferner betrifft die Erfindung Zusammensetzungen, die mindestens eine der Polysiloxanverbindungen zusammen mit mindestens einem weiteren für die Zusammensetzung üblichen Inhaltsstoff enthält.

Im folgenden sind einige typische Beispiele für derartige Zusammensetzungen gegeben, in denen die Polysiloxanverbindungen der Erfindung vorteilhaft verwendet werden können.

Typische Hilfsstoffe in derartigen Zusammensetzungen sind z.B. diejenigen Stoffe, die in A. Domsch: Die kosmetischen Präparate Bd. I u. II 4. Aufl. Verl. für chem. Industrie, H. Ziolkowsky KG, Augsburg sowie International Cosmetic Ingredient Dictionary and Handbook 7^{th} Ed. 1997 by J.A. Wenniger, G.N. McEwen Vol. 1-4 by The Cosmetic, Toiletry and Fragrance Association Washington DC bzw. unter http://www.cosmetic-world.com/inci /Incialf.htm beschrieben sind.

### Anionisches Shampoo

Das Formulierungsbeispiel ist als Rahmenrezeptur gedacht. Anionisches Shampoo enthalten üblicherweise die folgenden Komponenten, ohne auf diese beschränkt zu sein: Alkylsulfate, Alkylethersulfate, Natriumlaurylsulfat, Natriumlauryl-ethersulfat, Ammoniumlaurylsulfat, Ammoniumlauryl-ethersulfat, TEA-laurylsulfat, TEA-laurylethersulfat, Alkylbenzolsulfonate, α-Olefinsulfonate, Paraffinsulfonate, Sulfosuccinate, N-Acyltauride, Sulfat-glyceride, Sulfatierte Alkanolamide, Carboxylatsalze, N-acyl-Aminosäuresalze, Silicone, etc.

| Komponente | % |
|---|---|
| Ammoniumlaurylsulfat | 10.00 - 30.00 |
| Ammoniumlauryl-ethersulfat | 5.00-20.00 |
| Cocamidopropyl Betaine | 0.00 - 15.00 |
| Lauramid DEA | 0.00 - 5.00 |
| Cocamid Mea | 0.00 - 5.00 |
| Dimethicone Copolyol (Dimethylsiloxanglykolcopolymer) | 0.00 - 5.00 |
| Cyclopentasiloxane | 0.00-5.00 |
| Erfindungsgemäße Polysiloxanverbindung | 0.50 - 5.00 |
| Polyquaternium-10 | 0.00-2.00 |
| Konservierungsmittel | 0.00-0.50 |
| Duftstoffe | 0.00 - 5.00 |
| Entionisiertes Wasser | q.s. 100% |
| Natriumchlorid | q.s. |

### Nichtionisches Shampoo

Das Formulierungsbeispiel ist als Rahmenrezeptur gedacht. Nichtionische Shampoos enthalten üblicherweise die folgenden Komponenten, ohne auf diese beschränkt zu sein: Monoalkanolamide, Monoethanolamide, Monoisopropanolamide, Polyhydroxyderivative, Sucrosemonolaurat, Polyglycerinether, Aminoxide, Polyethoxylierte Derivative, Sorbitanderivative, Silicone, etc.

| Komponente | % |
|---|---|
| Lauramid DEA | 10.00 - 30.00 |
| Lauramid-Oxid | 5.00 - 20.00 |
| Cocamid Mea | 0.00 - 5.00 |
| Dimethicone Copolyol | 0.00 - 5.00 |
| Erfindungsgemäße Polysiloxanverbindung | 0.50 - 5.00 |
| Konservierungsmittel | 0.00 - 0.50 |
| Duftstoffe | 0.00 - 5.00 |
| Entionisiertes Wasser | q.s. 100% |
| Natriumchlorid | q.s. |

### Amphoteres Shampoo

Das Formulierungsbeispiel ist als Rahmenrezeptur gedacht. Formulierungen dieser Kategorie enthalten üblicherweise die folgenden Komponenten, ohne auf diese beschränkt zu sein:

N-Alkyl-iminodipropionate, N-Alkyl-iminopropionate, Aminosäuren, Aminosäurederivative, Amidobetaine, Imidazoliniumderivative, Sulfobetaine, Sultaine, Betaine, Silicone etc.

| Komponente | % |
|---|---|
| PEG-80-sorbitanlaurat | 10.00 - 30.00 |
| Lauroamphoglycinat | 0.00-10.00 |
| Cocamidopropyl-Hydroxysultain | 0.00-15.00 |
| PEG-150-distearat | 0.00-5.00 |
| Laurylether-13-carboxylat | 0.00-5.00 |
| Erfindungsgemäße Polysiloxanverbindung | 0.50-5.00 |
| Duftstoffe | 0.00 - 5.00 |
| Entionisiertes Wasser | q.s. 100% , |
| Natriumchlorid | q.s. |

### Kationisches Shampoo

Das Formulierungsbeispiel ist nur als Rahmenrezeptur gedacht. Formulierungen dieser Kategorie enthalten die folgenden Komponenten, ohne auf diese beschränkt zu sein: Bis-Quartäre Ammoniumverbindungen, Bis-(trialkylammoniumacetyl)diamine, Amidoamine, Ammonioalkylester, Silicone etc.

| Komponente | % |
|---|---|
| Laurylether-13-carboxylat | 10.00 - 30.00 |
| Isopropylmyristat | 5.00 - 20.00 |
| Cocamidopropyl-Betaine | 0.00 - 15.00 |
| Lauramid DEA | 0.00 - 5.00 |
| Cocamid MEA | 0.00 - 5.00 |
| Erfindungsgemäße Polysiloxanverbindung | 0.50 - 5.00 |
| Konservierungsmittel | 0.00 - 0.50 |
| Duftstoffe | 0.00 - 5.00 |
| Entionisiertes Wasser | q.s. 100% |
| Natriumchlorid | q.s. |

### Festiger

Das Formulierungsbeispiel ist nur als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone etc.

| Komponente | % |
|---|---|
| Ceteareth-20 | 0.10 - 10.00 |
| Steareth-20 | 0.10 - 10.00 |
| Stearyl-Alkohol | 0.10 - 10.00 |
| Stearamidopropyl-Dimethylamin | 0.00 - 10.00 |
| Dicetyldimonium-Chlorid | 0.00 - 10.00 |
| Erfindungsgemäße Polysiloxanverbindung | 0.50 - 5.00 |
| Cyclopentasiloxan | 0.00 - 5.00 |
| Dimethicone | 0.00 - 5.00 |
| Konservierungsmittel | 0.00 - 0.50 |
| Duftstoffe | 0.00 - 5.00 |
| Entionisiertes Wasser | q.s. 100% |

### "Clear Rinse -Off"-Festiger

Das Formulierungsbeispiel ist als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone etc.

| Komponente | % |
|---|---|
| Glycerin | 0.10-10.00 |
| Cetrimonium-Chlorid | 0.00-10.00 |
| Erfindungsgemäße Polysiloxanverbindung | 0.50 - 5.00 |
| Hydroxyethylcellulose | 0.00-5.00 |
| Konservierungsmittel | 0.00-0.50 |
| Duftstoffe | 0.00 - 5.00 |
| Entionisiertes Wasser | q.s. 100% |

### Schaumfestiger für Haare

Das Formulierungsbeispiel ist als Rahmenrezeptur gedacht. Formulierungen dieser Kategorie enthalten die folgenden Komponenten, ohne auf diese beschränkt zu sein: Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone Lösungsmittel, Ethanol, Isopropanol, Isoparaffinlösungsmittel, Butan, Propan, Isobutan, CFCs, Fluorierte Aereosoltreibmittel, Dimethylether, komprimierte Gase, etc.

| Komponente | % |
|---|---|
| Erfindungsgemäße Polysiloxanverbindung | 0.50 - 5.00 |
| Nonoxynol-15 | 0.00 - 2.00 |
| Nonoxynol-20 | 0.00 - 2.00 |
| Duftstoffe | 0.00 - 5.00 |
| Aereosoltreibmittel | 0.00 - 20.00 |
| Konservierungsmittel | 0.00 - 0.50 |
| Entionisiertes Wasser | q.s. 100% |

### Pumpspray (Festiger) für Haare

Das Formulierungsbeispiel ist nur als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone Lösungsmittel, Ethanol, Isopropanol, Isoparaffinlösungsmittel, etc.

| Komponente | % |
|---|---|
| Erfindungsgemäße Polysiloxanverbindung | 0.50 - 5.00 |
| Cyclomethicone | 0.00 - 80.00 |
| Ethanol | 0.00 - 80.00 |
| Konservierungsmittel | 0.00 - 0.50 |
| Duftstoffe | 0.00-5.00 |
| Entionisiertes Wasser | q.s. 100% |

### Festigerspray für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Fettsäuren, Fettsäureester, Ethoxylierte Fettsäuren, Ethoxylierte Fettsäureester, Fettalkohole, Ethoxylierte Fettalkohole, Glycole, Glycolester, Glycerin, Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone Lösungsmittel, Ethanol, Isopropanol, Isoparaffinlösungsmittel, Butan, Propan, Isobutan, CFCs, Fluorierte Aereosoltreibmittel, Dimethylether, Komprimierte Gase, etc.

| Komponente | % |
|---|---|
| Erfindungsgemäße Polysiloxanverbindung | 0.50-5.00 |
| Cyclomethicone | 0.00-80.00 |
| Ethanol | 0.00 - 50.00 |
| Aereosoltreibmittel | 0.00 - 50.00 |
| Konservierungsmittel | 0.00-0.50 |
| Duftstoffe | 0.00-5.00 |
| Entionisiertes Wasser | q.s. 100% |

### Gelfestiger für Haare

Das Formulierungsbeispiel ist als Rahmenrezeptur gedacht. Formulierungen dieser Kategorie enthalten üblichwerweise die folgenden Komponenten, ohne auf diese beschränkt zu sein:
Verdickungsmittel, Cellulosederivative, Acrylsäurederivative, Fixativ-Polymere, Konditionierungschemikalien, Glykole, Glykolester, Glycerin , Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Silicone, Lösungsmittel, Ethanol, Isopropanol, Isogaraffin-Lösungsmittel etc.

| Komponente | % |
|---|---|
| Erfindungsgemäße Polysiloxanverbindung | 0.50 - 5.00 |
| Hydroxyethylcellulose | 0.00 - 2.00 |
| Duftstoffe | 0.00 - 5.00 |
| Konservierungsmittel | 0.00 - 0.50 |
| Zitronensäure | 0.00 - 2.00 |
| Entionisiertes Wasser | q.s. 100% |

### Styling Gel für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Fixativ-Polymere, Lacke, Acrylisäurederivative, Cellulosederivative, Vinylderivative, Konditionierungschemikalien, Glykole, Glykolester, Glycerin , Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone, Lösungsmittel, Ethanol, Isopropanol, Isoparaffin-Lösungsmittel etc.

| Komponente | % |
|---|---|
| Erfindungsgemäße Polysiloxanverbindung | 0.50 - 5.00 |
| Fixative | 0.10-10.00 |
| Hydroxyethylcellulose | 0.00 - 2.00 |
| Duftstoffe | 4.00 - 5.00 |
| Zitronensäure | 0.00 - 2.00 |
| Entionisiertes Wasser | q.s. 100% |

### Styling Spray für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Fixativ-Polymere, Lacke, Vinylderivative, Fettsäuren, Fettsäureester, Ethoxylierte Fettsäuren, Ethoxylierte Fettsäureester, Fettalkohole, Ethoxylierte Fettalkohole, Glykole, Glykolester, Glycerin, Glycerin-Ester, Lanolin, Lanolinderivative, Mineral öl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone, Lösungsmittel, Ethanol, Isopropanol, Isoparaffinlösungsmittel, Butan, Propan, Isobutan, CFCs, Fluorierte Aerosoltreibmittel, Dimethylether, Komprimierte Gase, etc.

| Komponente | % |
|---|---|
| Erfindungsgemäße Polysiloxanverbindung | 0.50 - 5.00 |
| Cyclomethicone | 0.00 - 80.00 |
| Fixative | 0.10 - 10.00 |
| Ethanol | 0.00 - 50.00 |
| Aerosoltreibmittel | 0.40 - 50.00 |
| Konservierungsmittel | 0.00 - 0.50 |
| Duftstoffe | 0.00 - 5.00 |
| Entionisiertes Wasser | q.s. 100% |

### Pumpspray (Styling) für Haare

Das Formulierungsbeispiel ist als Rahmenrezeptur gedacht. Formulierungen dieser Kategorie enthalten üblicherweise die folgenden Komponenten, ohne auf diese beschränkt zu sein:
Vinylderivative, Fixativ-Polymere, Lacke, Fettsäuren, Fettsäureester, Ethoxylierte Fettsäuren, Ethoxylierte Fettsäureester, Fettalkohole, Ethoxylierte Fettalkohol, Glykole, Glykolester, Glycerin , Glycerinester, Lanolin, Lanolinderivative, Mineralöl, Petrolatum, Lecithin, Lecithinderivative, Wachse, Wachsderivative, Kationische Polymere, Proteine, Proteinderivative, Aminosäuren, Aminosäurederivative, Feuchthaltemittel, Verdickungsmittel, Silicone, Lösungsmittel, Ethanol, Isopropanol, Isoparaffinlösungsmittel, Butan, Propan, Isobutan, CFCs, Fluorierte Aerosoltreibmittel, Dimethylether, komprimierte Gase, etc.

| Komponente | % |
|---|---|
| Erfindungsgemäße Polysiloxanverbindung | 0.50-5.00 |
| Fixative | 0.10-10.00 |
| Cyciomethicone | 0.00 - 80.00 |
| Ethanol | 0.00-50.00 |
| Konservierungsmittel | 0.00-0.50 |
| Duftstoffe | 0.00 - 5.00 |
| Entionisiertes Wasser | q.s. 100% |

Die Verwendung der erfindungsgemäßen Polysiloxanderivate führt bei Anwendung im Haarkosmetikbereich zu günstigen Effekten hinsichtlich Festigung, Glanz, Fixierung (Halt), Körper, Volumen, Feuchtigkeitsregulierung, Farbretention, Schutz vor Umwelteinflüssen (UV, Salzwasser u.s.w.), Wiederformbarkeit, Antistatischen Eigenschaften, Färbbarkeit etc.

### Beispiele

Die nachfolgenden Beispiele sollen die Erfindung näher erläutern, ohne sie jedoch einzuschränken.

### Beispiel 1

1a) In einem 1 Liter Dreihalskolben wurden 24 g Wasser und 4,18 g (0,048 mol tertiäre Aminogruppen) N,N,N',N'-Tetramethyl-1,6-hexandiamin und 12,77 g (0,012 mol primäre Aminogruppen ) eines unter dem Handelsnamen Jeffamin® ED 2003 erhältlichen Alkylenoxidderivates der Struktur

H₂NCH(CH₃)CH₂[OCH₂CH(CH₃)]ₐ(OCH₂CH₂)_{38,7}[OCH₂CH(CH₃)]_{b}NH₂

mit a + b = 6
bei Raumtemperatur vorgelegt. Innerhalb von 5 Minuten wurden 12,0 g (0,03 mol) Dodecansäure in Form einer 50 %igen Lösung in 2-Propanol und 1,8 g (0,03 mol) Essigsäure zugesetzt. Nach Erwärmung des Ansatzes auf 50 °C wurden innerhalb von 30 Minuten 194,1 g (0,06 mol Epoxygruppen) eines Epoxysiloaans der durchschnittlichen Zusammensetzung 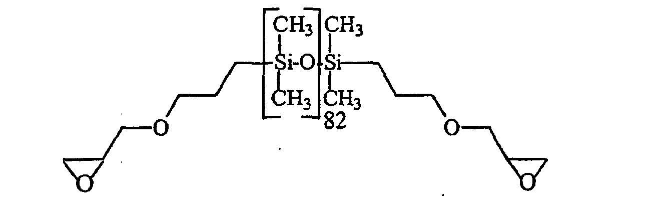 und 30 ml 2-Propanol zugetropft. Die gelbe, trübe Mischung wurde 6 Stunden auf Rückflußtemperatur erhitzt. Nach Entfernung aller bis 100 °C und bei 2mmHg im Vakuum flüchtigen Bestandteile werden 209 g eines beigen, trüben Materials der Struktur 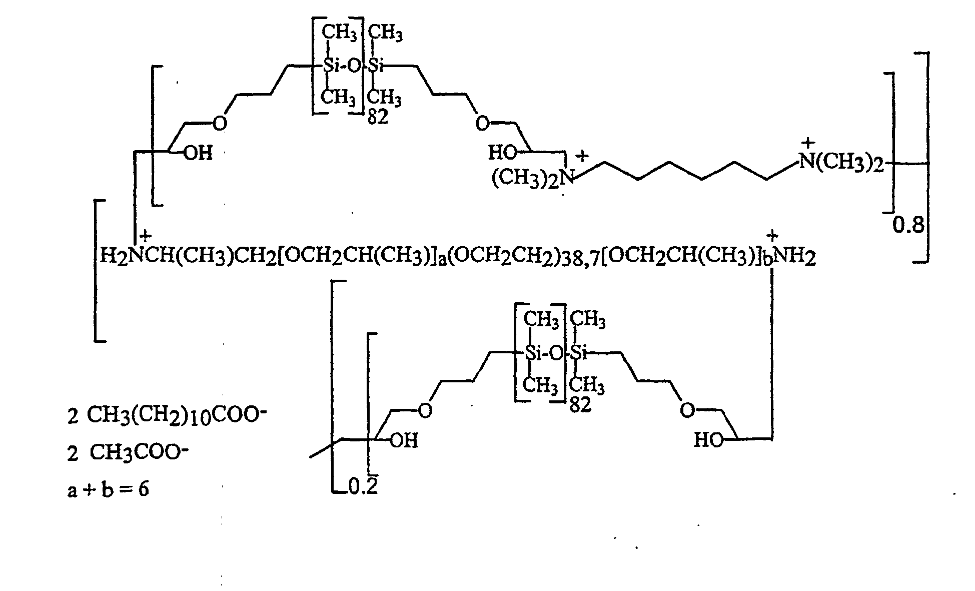 erhalten. Dabei ist die o.g. Formel so zu verstehen, dass es sich um ein statistisches Copolymer handelt, worin das molare Verhältnis der beiden Amine 0,8 zu 0,2 beträgt.

### ¹³C-NMR:

| **Substruktur** | **Shift (ppm)** |
|---|---|
| -CH(OH)-CH₂-N⁺[(CH₃)₂]-CH₂-CH₂- | 65,4 |
| -CH(OH)-CH₂-N⁺[(CH₃)₂]-CH₂-CH₂- | 64,1 |
| -CH(OH)-CH₂-N⁺[(CH₃)₂]-CH₂-CH₂- | 52,3/52,5 |
| -CH(OH)-CH₂-N⁺[(CH₃)₂]-CH₂-CH₂- | 63,8 |
| -CH(OH)-CH₂-N⁺[(CH₃)₂]-CH₂-CH₂- | 23,4 |
| -CH₂-O-CH₂- | 70,6 |

### Beispiel 1a)

In analoger Weise wie bei Beispiel 1 wird ein Trimethylaminterminiertes Polysiloxan durch Umsetzung von
0,08 mol Tetramethylhexamethylendiamin,
0,01 mol Jeffamine ED 2003,
0,02 mol Trimethylamin,
0,1 mol eines Diepoxids der Formel 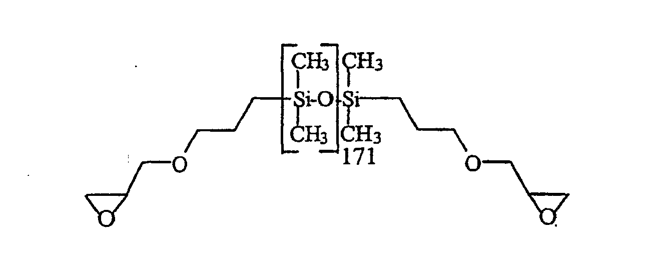 0,05 mol Dodecansäure, und
0,05 mol Essigsäure
umgesetzt und das trimethylaminterminierte Polysiloxan isoliert.

### Beispiel 2

Zum Nachweis der Eignung als waschbeständiger, hydrophiler Weichmacher wurde weißer Baumwolljersey der nachfolgend beschriebenen Behandlung mit einer auf dem quartemären Ammoniumsalz gemäß Beispiel 1 beruhenden Formulierung unterworfen. Zum Vergleich wurde ein kommerziell erhältlicher hydrophiler Weichmacher HSSD Magnasoft® von OSi Specialities benutzt. Es wurden zunächst folgende klare Stammformulierungen hergestellt:

| Beispiel 1 | Vergleichsbeispiel Magnasoft® HSSD |
|---|---|
| 20,0 g Beispiel 1 | 20,0 g Siloxanweichmacher |
| 0,4 g Essigsäure | 0,2 g Essigsäure |
| 74,6 g dest. Wasser | 79,8 g dest. Wasser |
| 5,0 g Renex 36® (Henkel) | |

20 g dieser Stammformulierungen wurden in 980 g destilliertem Wasser gelöst. Anschließend erfolgt mit diesen effektiv 0,4 % Siloxanwirkstoff enthaltenden Formulierungen eine Ausrüstung von 60 cm x 90 cm großen und 87 g schweren Baumwolljerseystücken im Foulardverfahren. Hierzu wurde das Baumwollmaterial 5 bis 10 Sekunden vollständig in die jeweilige Formulierung eingetaucht und nach Zwangsapplikation bei 120 °C, 3 Minuten lang getrocknet.

Nachfolgend wurden die Lappen geteilt und jeweils eine Hälfte fünf maschinellen Waschzyklen in Gegenwart eines Feinwaschmittels (1,7 g Waschmittel/ Liter Waschflotte) unterzogen. Jeder Waschzyklus dauerte 25 Minuten, die Waschtemperatur betrug 40 °C.

An den ungewaschenen und gewaschenen Textilstücken wurde die Hydrophilie (Einziehzeit eines Wassertropfens in Sekunden) bestimmt und zusätzlich von 10 Testpersonen der Griff bewertet.

| | Hydrophilie (s) | Griff |
|---|---|---|
| Beispiel 1 ausgerüstet; ungewaschen | <3 | Glatt, flach, weich |
| Beispiel 1 ausgerüstet; 5 x gewaschen | <2 | Glatt, flach, mittelweich |
| Vergleichsbeispiel; ungewaschen | 1 | Glatt, flach |
| Vergleichsbeispiel; 5 x gewaschen | 1 | Hart |

Die Ergebnisse zeigten, daß das erfindungsgemäß ausgerüstete Textilmaterial auch nach 5 Waschzyklen noch über die gewünschte Eigenschaftskombination aus Hydrophilie, ausgedrückt durch eine sehr kurze Tropfeneinziehzeit, und dem silicontypischem Griff verfügte.

### Beispiel 3

Zum Nachweis der weichmachenden Eigenschaften als interner Weichmacher während des Waschprozesses wurden gebleichte und an der Oberfläche nicht weiter ausgerüstete Baumwollstreifen einem Waschprozeß in Gegenwart von Ariel Futur®, bentonithaltigem Dash 2 in 1® sowie des in Beispiel 1 beschriebenen Verbindungen unterworfen. Es wurden folgende Randbedingungen eingehalten.

| | Streifen 1 | Streifen 2 | Streifen 3 |
|---|---|---|---|
| Streifengewicht (g) | 12,94 | 13,00 | 13,10 |
| Wassermenge (ml) | 641 | 653 | 670 |
| Detergenz | 0,64g Ariel Futur® | 0,65g Ariel Futur® | 0,66g Dash 2 in 1® |
| Polysiloxanverbindung Bsp. 1 | 0,2 g | - | - |
| Note Ø | 1,3 | 2,9 | 1,8 |

Das Wasser wurde auf 60 °C erhitzt, die Detergenzien und im Falle des Baumwollstreifens 1 zusätzlich die Verbindung gemäß Beispiel 1gelöst. Anschließend wurden die Baumwollstreifen in diesen Lösungen für 30 Minuten gewaschen. Nachfolgend wurden die Streifen jeweils fünfmal mit 600 ml Wasser gespült und abschließend 30 Minuten bei 120 °C getrocknet.

16 Testpersonen bewerteten die drei Baumwollstreifen auf die Weichheit des Griffs hin, wobei die Note 1 dem weichesten Streifen und die Note 3 den als am härtesten empfundenen Streifen zugeteilt wurde.

Im Ergebnis der Bewertung erhielt der Baumwollstreifen 1 die Durchschnittsnote 1,3. Der Baumwollstreifen 2 wurde durchschnittlich mit 2,9 und der Bentonit behandelte Streifen 3 mit 1,8 bewertet.

## Patentansprüche

1. Polysiloxanpolymere mit den Wiederholungseinheiten 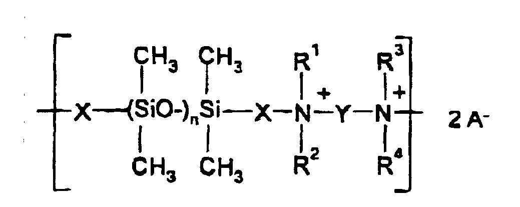 und 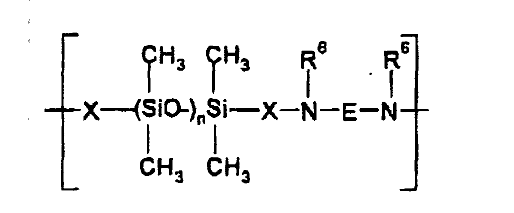 worin
X ein zweiwertiger Kohlenwasserstoffrest mit mindestens 4 Kohlenstoffatomen ist, der eine Hydroxylgruppe aufweist und der durch ein Sauerstoffatom unterbrochen sein kann, und die Gruppen X in den Wiederholungseinheiten gleich oder verschieden sein können,
Y ein zweiwertiger Kohlenwasserstoffrest mit mindestens 2 Kohlenstoffatomen ist, der eine Hydroxylgruppe aufweisen und der durch ein oder mehrere Sauerstoff- oder Stickstoffatome unterbrochen sein kann,
R¹, R², R³ und R⁴ gleich oder verschieden sind und Alkylreste mit 1 bis 4 Kohlenstoffatomen oder Benzylreste bedeuten oder jeweils die Reste R¹ und R³ oder R² und R⁴ Bestandteile eines verbrückenden Alkylenrestes sein können,
R⁶ H oder ein Alkylrest mit 1 bis 20 Kohlenstoffatomen ist, der sauerstoffsubstituiert sein kann,
E die Struktur -B-O-(EOx)ᵥ(POx)_{w}-B-, worin Eox eine Ethylenoxideinheit und PO_{X} eine Propylenoxideinheit ist, und
B gradkettiges oder verzweigtes C₂ bis C₆ Alkylen,
v 0 bis 200,
w 0 bis 200,
v+w ≥ 1 entspricht,
n 2 bis 1000, worin die n in den Wiederholungseinheiten gleich oder verschieden sein können,
A⁻ ein anorganisches oder organisches Anion bedeutet.

2. Polysiloxanpolymere nach Anspruch 1, **dadurch gekennzeichnet, daß** es sich um lineare oder cyclische Polysiloxanpolymere handelt.

3. Palysiloxanpolymere nach Anspruch 1 oder 2, **dadurch gekennzeichnet, daß** es sich um lineare Polysiloxanpolymere handelt, die terminale Gruppen enthalten, ausgewählt aus 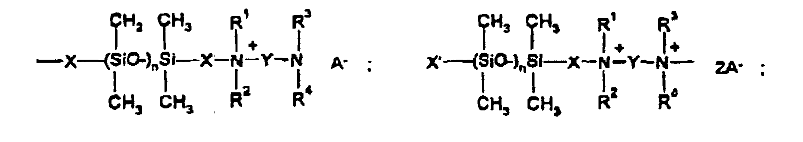 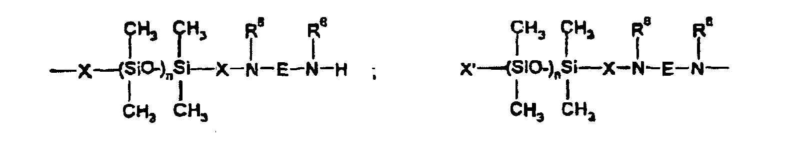 und/oder 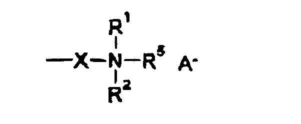 worin X, R¹, R², R³, R⁴, R⁶, Y, E, A⁻ und n wie oben definiert sind,
X' ein Kohlenwasserstoffrest mit mindestens 4 Kohlenstoffatomen ist, der eine Epoxygruppe oder eine mit Alkoholen, Wasser oder Aminen geöffnete Epoxygruppe aufweist und der durch ein Sauerstoffatom unterbrochen sein kann, und
R⁵ ein Alkylrest mit 1 bis 20 Kohlenstoffatomen ist, und
wobei die endstindigen Gruppen X in den terminalen Gruppen jeweils an die endständigen Stickstoffatome der Wiederholungseinheiten und die endständigen Stickstoffatome in den terminalen Gruppen jeweils an die endständigen Gruppen X der Wiederholungseinheiten binden.

4. Polysiloxanpolymere nach irgend einem der Ansprüche 1 bis 3,
**dadurch gekennzeichnet, daß** es cyclische polyquartemäre Polysiloxanpolymere der allgemeinen Formel (I) 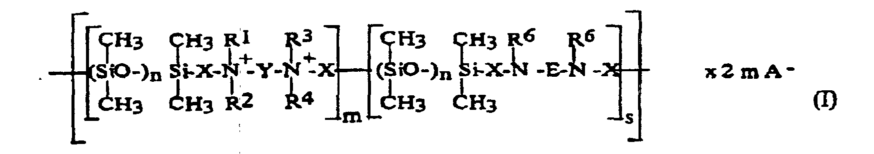 und/oder linearen Verbindungen der allgemeinen Formel (II) 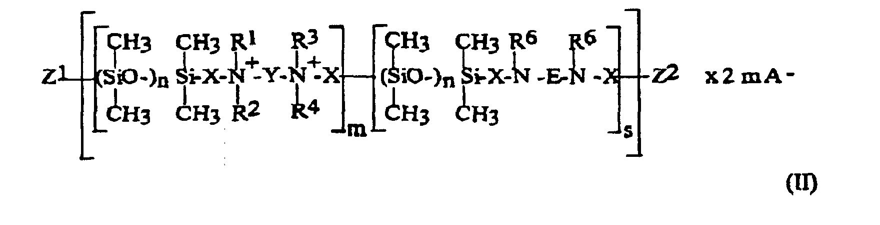 sind, worin
X ein zweiwertiger Kohlenwasserstoffrest mit mindestens 4 Kohleastoffetomen ist, der eine Hydroxylgruppe aufweist und der durch ein Sauerstoffatom unterbrochen sein kann,
Y ein zweiwertiger Kohlenwasserstoffrest mit mindestens 2 Kohlenstoffatomen ist, der eine Hydroxylgruppe aufweisen und der durch ein oder mehrere Sauerstoff- oder Stickstoffatome unterbrochen sein kann,
Z¹ ein H, OH, ein Alkyl-, ein Epoxy- oder ein Alkoxyrest ist, oder die Bedeutung eines Kohtenwasserstoffrestes mit mindestens 4 Kohlenstoffatomen hat, der eine oder mehrere Hydroxylgruppe(n) aufweist und durch eine oder mehrere Sauerstoffatome unterbrochen sein kann oder die Bedeutung des Restes 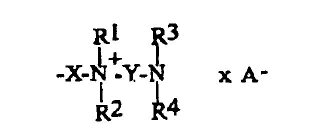 oder 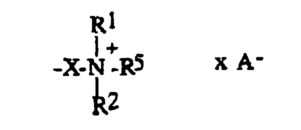 hat, wobei R⁵ ein Alkylrest mit 1 bis 20 KohlenstoMtumen ist,
Z² die Gruppe 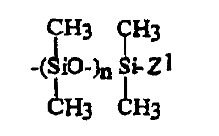
R¹, R², R³ und R⁴ gleich oder verschieden sind und Alkylreste mit 1 bis 4 Kohlenstoffatomen oder Benzylreste bedeuten oder jeweils die Reste R¹ und R³ oder R² und R⁴ Bestandteile eines verbrilckenden Alkylenrestes sein können,
R⁶ H oder ein Alkylrest mit 1 bis 20 Kohlenstoffatomen, der sauerstoffsubstituiert sein kann,
E die Struktur -B-(OCH₂CH₂)ᵥ(OCH₂CH(CH₃))_{w}-O-B- mit
B gradkettig oder verzweigt C₂ bis C₆ Alkylen,
v 0 bis 200,
w 0 bis 200,
v+w ≥ 1 entspricht,
A⁻ ein anorganisches oder organisches Anion
n 5 bis 200
m ganze Zahl ≥ und
s ganze Zahl ≥ 1
bedeuten.

5. Polysiloxanpolymere nach irgend einem der Ansprüche 1 bis 4, **dadurch gekennzeichnet, dass** n = 5 bis 82 ist.

6. Polysiloxanpolymere nach irgend einem der Ansprüche 1 bis 5, **dadurch gekennzeichnet, dass** n = 5 bis 20 ist.

7. Polysiloxanpolymere nach irgend einem der Ansprüche 1 bis 7, **dadurch gekennzeichnet, daß** X ein Rest aus der Gruppe 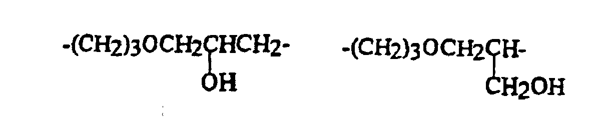 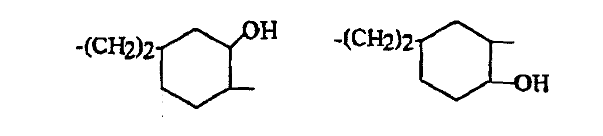 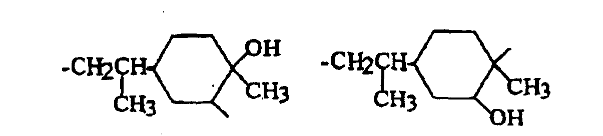 darstellt, Y ein Rest -(CH₂)ₒ- , mit o von 2 bis 6 ist, die Gruppen R¹, R², R³, R⁴ Methylreste sind, und R⁶ Wasserstoff, -CH₂CH₂OH und -CH₃ ist.

8. Potysitoxanpolymere nach irgend einem der Ansprüche 1 bis 7, **dadurch**
**dadurch gekennzeichnet, daß** das Strukturelement B durch -CH₂CH₂- und -CH₂CH(CH-₃)- Einheiten dargestellt wird.

9. Polysiloxanpolymere nach irgend einem der Ansprüche 1 bis 8, **dadurch gekennzeichnet, daß** Y -(CH₂)ₒ- , mit o von 2 bis 6 bedeutet.

10. Polysiloxanpolymere nach irgend einem der Ansprüche 1 bis 9, **dadurch gekennzeichnet, daß** R¹, R², R³, R⁴ Methyl ist.

11. Polysiloxanpolymere nach irgend einem der Ansprüche 1 bis 10, **dadurch gekennzeichnet, daß** R⁶ Wasserstoff, -CH₂CH₂OH und -CH₃ ist.

12. Polysiloxanpolymere nach irgend einem der Ansprüche 1 bis 11, **dadurch gekennzeichnet, daß** B für -CH₂CH₂- und -CH₂CH(CH₃)- Einheiten steht.

13. Polysiloxanpolymere nach irgend einem der Ansprüche 1 bis 12, **dadurch gekennzeichnet, daß** v 0 bis 100 bedeutet.

14. Polysiloxanpolymere nach irgend einem der Ansprüche 4 bis 13, **dadurch dadurch gekennzeichnet, daß** m/(m+s) x100 = 0,1 bis 99,9 %.

15. Polysiloxanpolymere nach irgend einem der Ansprüche 1 bis 14, **dadurch gekennzeichnet, daß** w 0 bis 100 bedeutet.

16. Polysiloxanpolymere nach irgend einem der Ansprüche 1 bis 15, **dadurch gekennzeichnet, dass** als Anionen A⁻ physiologisch vertretbare anorganische Reste aus der Gruppe bestehend aus Chlorid, Bromid, Hydrogensulfat, Sulfat, oder organische Reste, aus der Gruppe bestehend aus Acetat, Propionat, Octanoat, Decanoat, Dodecanoat, Tetradecanoat, Hexadecanoat, Octadecanoat, Oleat verwendet werden.

17. Polysiloxanpolymere nach irgend einem der Ansprüche 1 bis 16, **dadurch gekennzeichnet, daß** die Verbindungen in protonierter Form als Aminsalze vorliegen.

18. Verfahren zur Herstellung von Polysiloxanpolymeren nach irgend einem der Ansprüche 1 bis 17, **dadurch gekennzeichnet, dass** man Bisepoxid-terminierte Polysiloxane der Formel 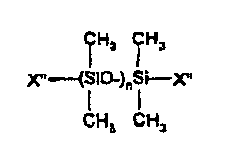 worin X" ein zweiwertiger Kohlenwasserstoffrest mit mindestens 4 Kohlenstoffatomen ist, der eine Epoxygruppe aufweist und der durch ein Sauerstoffatom unterbrochen sein kann, mit Bisaminen der Formeln 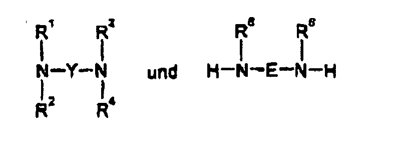 in geeigneter Reihenfolge, gegebenenfalls unter Zusatz eines Monoamins der Formel 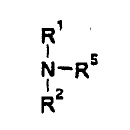 umsetzt, worin die Substituenten wie oben definiert sind.

19. Polysiloxanpolymere nach irgend einem der Ansprüche 1 bis 17 zu Verwendung als hydrophile Weichmacher auf Basis quarternärer Ammoniumgruppen enthaltender Siloxane.

20. Verwendung der Polysiloxanpolymere nach irgend einem der Ansprüche 1 bis 17 und 19, als waschbeständiger hydrophiler Weichmacher.

21. Verwendung der Polysiloxanpolymere nach Anspruch 20, als waschbeständiger hydrophiler Weichmacher für die textile Erstausrüstung.

22. Verwendung der Polysiloxanverbindungen nach irgend einem der Ansprüche 1 bis 17, in kosmetischen Formulierungen für die Haut- und Haarpflege, in Polituren für die Behandlung und Ausrüstung harter Oberflächen, in Formulierungen zum Trocknen von Automobilen und anderen harten Oberflächen nach maschinellen Wischen, zur Ausrüstung von Textilen und Textilfasern, als separate Weichmacher nach dem Waschen von Textilien mit nichtionogenen oder anionischen/nichtionogen Detergenzien-formulierungen, als Weichmacher in auf nichtionischen oder anionischen/nichtionischen Tensiden beruhenden Formulierungen zur Textilwäsche.

## Claims

1. Polysiloxane polymers with the repetition units 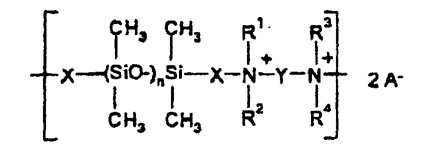 and 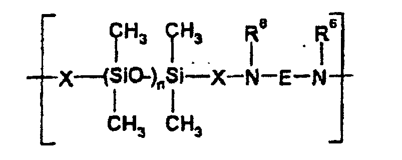 wherein
X is a divalent hydrocarbon radical with at least 4 carbon atoms, which features a hydroxyl group and which can be interrupted by an oxygen atom, and the X groups can be identical or different in the repetition units,
Y is a divalent hydrocarbon radical with at least 2 carbon atoms, which may feature a hydroxyl group and be interrupted by one or more oxygen or nitrogen atoms,
R¹, R², R³ and R⁴ are identical or different and represent alkyl radicals with 1 to 4 carbon atoms or benzyl radicals, or in each case the R¹ and R³ radicals or the R² and R⁴ radicals may be part of a bridging alkylene radical,
R⁶ is H or an alkyl radical with 1 to 20 carbon atoms, which may be oxygen-substituted,E is the structure -B-O-(EOₓ)ᵥ(POₓ)_{w}-B-, wherein EOₓ is an ethylene oxide unit and POₓ a propylene oxide unit, and
B is a straight chain or branched C₂ to C₆ alkylene, v is 0 to 200, w is 0 to 200, v+w corresponds to ≥ 1, n is 2 to 1000, wherein the n's in the repetition units may be identical or different,
A⁻ means an inorganic or organic anion.

2. Polysiloxane polymers according to Claim 1, **characterized in that** they are linear or cyclic polysiloxane polymers.

3. Polysiloxane polymers according to Claim 1 or 2, **characterized in that** they are linear polysiloxane polymers containing terminal groups selected among 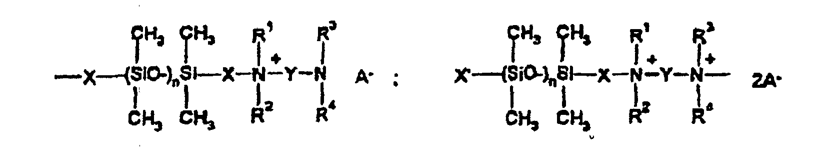 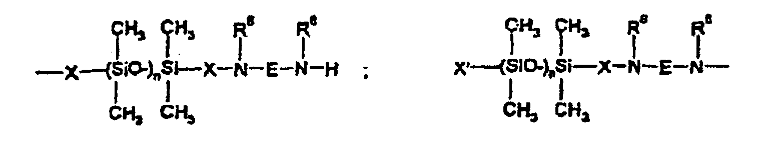 and/or 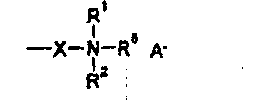 wherein X, R¹, R², R³, R⁴, R⁶, Y, E, A⁻ and n are defined the same as above,
X' is a hydrocarbon radical with at least 4 carbon atoms, which features an epoxy group or an epoxy group opened with alcohols, water or amines and which may be interrupted by an oxygen atom, and R⁵ is an alkyl radical with 1 to 20 carbon atoms, and
wherein the terminal X groups in the terminal groups bind to the terminal nitrogen atoms of the repetition units in each case, and the terminal nitrogen atoms in the terminal groups bind in each case to the terminal X groups of the repetition units.

4. Polysiloxane polymers according to any of Claims 1 to 3, **characterized in that** they are cyclic, polyquarternary polysiloxane polymers of the general formula (I) 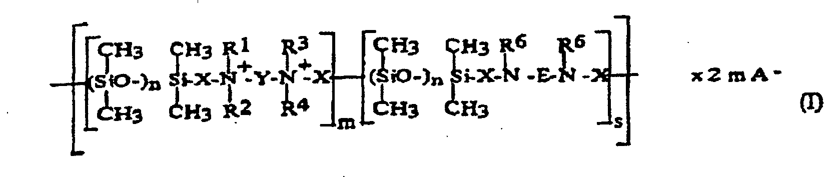 and/or linear compounds of the general formula (II) 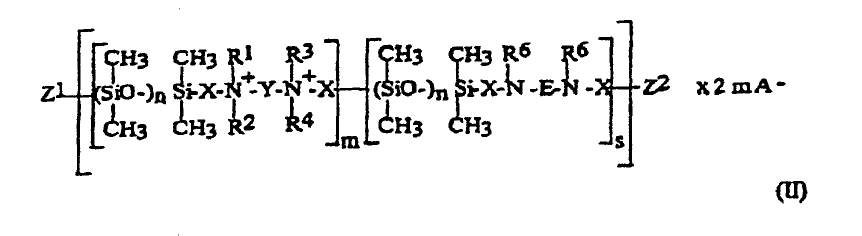 wherein
X is a divalent hydrocarbon radical with at least 4 carbon atoms, which features a hydroxyl group and may be interrupted by an oxygen atom,
Y is a divalent hydrocarbon radical with at least 2 carbon atoms, which may feature a hydroxyl group and may be interrupted by one or more oxygen or nitrogen atoms,
Z¹ is a H, OH, an alkyl, an epoxy or an alkoxy radical, or has the meaning of a hydrocarbon radical with at least 4 carbon atoms, which features one or more hydroxyl group(s) and can be interrupted by one or more oxygen atoms, or has the meaning of the radical 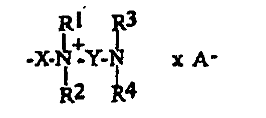 or 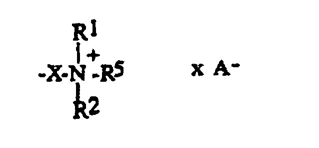 wherein R⁵ is an alkyl radical with 1 through 20 carbon atoms,
Z² is the group 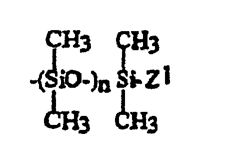 R¹, R², R³ and R⁴ are identical or different and represent alkyl radicals with 1 to 4 carbon atoms or benzyl radicals, or the R¹ and R³ radicals or the R² and R⁴ radicals in each case may be part of a bridging alkylene radical,
R⁶ is H or an alkyl radical with 1 to 20 carbon atoms, which may be oxygen substituted,
E is the structure -B-(OCH₂CH₂)ᵥ(OCH₂CH)CH₃))_{w}-O-B-, wherein
B is straight chain or branched chain C₂ to C₆ alkylene,
v is 0 to 200,
w is 0 to 200,
v+w corresponds to ≥ 1,
A⁻ means an inorganic or organic anion,
n is 5 to 200,
m is a whole number ≥ 1 and
s is a whole number ≥ 1.

5. Polysiloxane polymers according to any of Claims 1 to 4, **characterized in that** n=5 to 82.

6. Polysiloxane polymers according to any of Claims 1 to 5, **characterized in that** n=5 to 20.

7. Polysiloxane polymers according to any of Claims 1 to 7, **characterized in that** X is a radical from the group 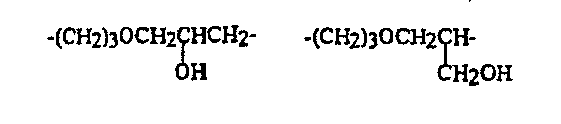 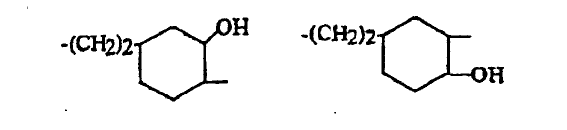 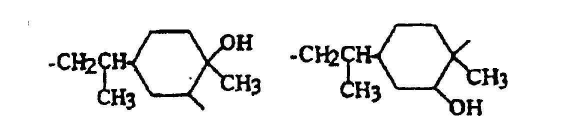 Y is a radical -(CH₂)ₒ-, where o is 2 to 6, the groups R¹, R², R³, R⁴ are methyl radicals, and R⁶ is hydrogen, -CH₂CH₂OH and -CH₃.

8. Polysiloxane polymers according to any of Claims 1 to 7, **characterized in that** the structural element B is represented by -CH₂CH₂- and -CH₂CH(CH₃) - units.

9. Polysiloxane polymers according to any of Claims 1 to 8, **characterized in that** Y signifies -(CH₂)ₒ-, where o is 2 to 6.

10. Polysiloxane polymers according to any of Claims 1 to 9, **characterized in that** R¹, R², R³, R⁴ is methyl.

11. Polysiloxane polymers according to any of Claims 1 to 10, **characterized in that** R⁶ is hydrogen, -CH₂CH₂OH and -CH₃.

12. Polysiloxane polymers according to any of Claims 1 to 11, **characterized in that** B represents -CH₂CH₂₋and -CH₂CH(CH₃)- units.

13. Polysiloxane polymers according to any of Claims 1 to 12, **characterized in that** v means 0 to 100.

14. Polysiloxane polymers according to any of Claims 4 to 13, **characterized in that** m/(m+s)×100=0.1 to 99.9%.

15. Polysiloxane polymers according to any of Claims 1 to 14, **characterized in that** w stands for 0 to 100.

16. Polysiloxane polymers according to Claims 1 to 15, **characterized in that** physiologically tolerable inorganic radicals from among the group consisting of chloride, bromide, hydrogen sulphate, sulphate and/or organic radicals from among the group consisting of acetate, propionate, octanoate, decanoate, dodecanoate, tetradecanoate, hexadecanoate, octadecanoate, oleate are used as anions A⁻.

17. Polysiloxane polymers according to any of Claims 1 to 16, **characterized in that** the compounds are present in a protonated form as amine salts.

18. Method for the production of polysiloxane polymers according to any of Claims 1 to 17, **characterized in that** bis epoxide-terminated polysiloxanes of the formula 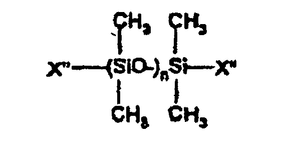 wherein X" is a divalent hydrocarbon radical with at least 4 carbon atoms, which features an epoxy group and may be interrupted by an oxygen atom, are converted with bis amines of the formulae 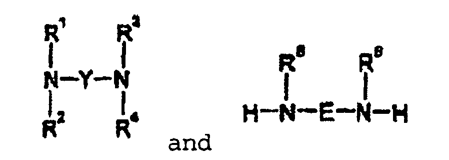 in the appropriate order, possibly by adding a monoamine of the formula 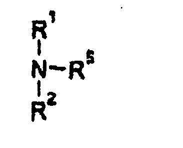 wherein the substituents are defined the same as above.

19. Polysiloxane polymers according to any of Claims 1 to 17 for use as hydrophilic softeners on the basis of quarternary siloxanes containing ammonium groups.

20. Use of the polysiloxane polymers according to any of Claims 1 to 17 and 19 as a wash-resistant hydrophilic softener.

21. Use of the polysiloxane polymers according to Claim 20 as a wash-resistant hydrophilic softener for the initial finishing of textiles.

22. Use of the polysiloxane compounds according to any of Claims 1 to 17 in cosmetic skin and hair care formulations, in polishes for the treatment and finishing of hard surfaces, in formulations for drying cars and other hard surfaces after machine washing, for finishing textiles and textile fibres, as a separate softener after laundering textiles with non-ionogenic or anionic/non-ionogenic detergent formulations, as a softener in formulations for laundering textiles, which are based on non-ionic or anionic/non-ionic surfactants.

## Revendications

1. Polymères de polysiloxane avec les unités périodiques 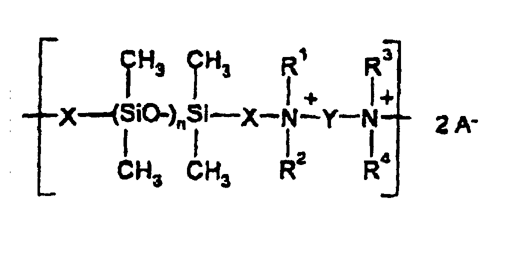 et 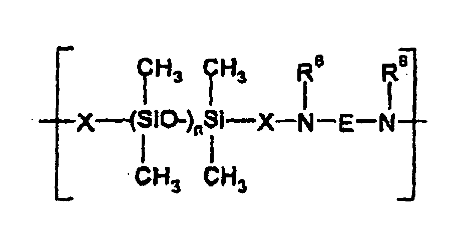 dans lesquelles
X est un radical hydrocarbure divalent avec au moins 4 atomes de carbone, qui comporte un groupe hydroxyle et qui peut être interrompu par un atome d'oxygène, et dans lesquelles les groupes X peuvent être identiques ou différents d'une unité périodique à l'autre,
Y est un radical hydrocarbure divalent avec au moins 2 atomes de carbone, qui comporte un groupe hydroxyle et qui peut être interrompu par un ou plusieurs atomes d'oxygène ou d'azote,
R¹, R², R³ et R⁴
sont identiques ou différents et signifient des radicaux alkyles avec 1 à 4 atomes de carbone ou des radicaux benzyliques, ou dans lesquelles R¹ et R³ ou R² et R⁴ respectivement peuvent être des composants d'un radical alkylène à effet de pontage,
R⁶ est un H ou un radical alkyle avec 1 à 20 atomes de carbone qui peut être substitué par des oxygènes,
E a la structure -B-O-(Eox)ᵥ(Pox)_{w}-B-, dans laquelle Eox est une unité d'oxyde d'éthylène et Pox est une unité d'oxyde de propylène et
B est un alkylène en C₂ à C₆ linéaire ou ramifié,
v 0 à 200
w 0 à 200
v+w correspond à ≥ 1,
n 2 à 1000, sachant que les n peuvent être identiques ou différents d'une unité périodique à l'autre,
A' signifie un anion inorganique ou organique.

2. Polymères de polysiloxane selon la revendication 1, **caractérisés en ce qu'**il s'agit de polymères de polysiloxane linéaires ou cycliques.

3. Polymères de polysiloxane selon la revendication 1 ou 2, **caractérisés en ce qu'**il s'agit de polymères de polysiloxane linéaires qui contiennent des groupes de terminaison choisis parmi 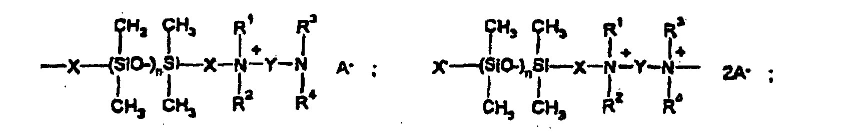 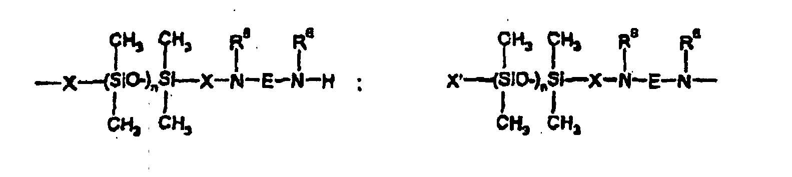 et/ou 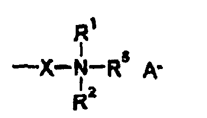 dans lesquels X, R¹, R², R³, R⁴, R⁶ Y, E, A' et n sont définis comme ci-dessus,
X' est un radical hydrocarbure avec au moins 4 atomes de carbone qui comporte un groupe époxyde ou un groupe époxyde ouvert par des alcools, de l'eau ou des amines et qui peut être interrompu par un atome d'oxygène et
R⁵ est un radical alkyle avec 1 à 20 atomes de carbone, et
sachant que les groupes finals X dans les groupes de terminaison s'attachent respectivement aux atomes d'azote finals des unités périodiques et que les atomes d'azote finals dans les groupes de terminaison s'attachent respectivement aux groupes finals X des unité périodiques.

4. Polymères de polysiloxane selon l'une quelconque des revendications 1 à 3, **caractérisés en ce qu'**ils sont des polymères de polysiloxane polyquaternaires de la formule générale (I) 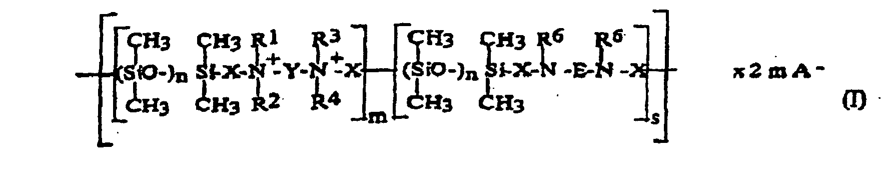 et/ou des composés linéaires de la formule générale (II) 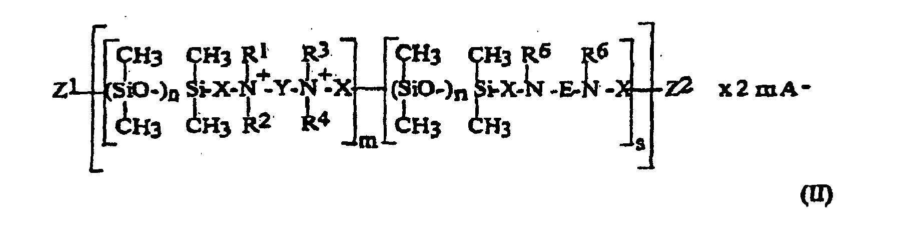 dans lesquelles
X
comporte un est un radical hydrocarbure divalent avec au moins 4 atomes de carbone, qui groupe hydroxyle et qui peut être interrompu par un atome d'oxygène,
Y est un radical hydrocarbure divalent avec au moins 2 atomes de carbone, qui comporte un groupe hydroxyle et qui peut être interrompu par un ou plusieurs atomes d'oxygène ou d'azote,
Z¹ est un H un OH, un radical alkyle, époxyde ou alcoxyle, ou a la signification d'un radical hydrocarbure avec au moins 4 atomes de carbone, qui comporte un ou plusieurs groupes hydroxyles et peut être interrompu par un ou plusieurs atomes d'oxygène, ou qui a la signification du radical 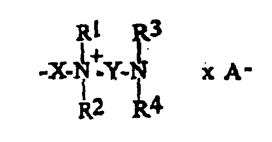 ou 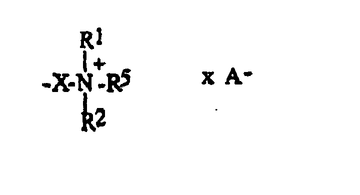 dans lesquels R⁵ est un radical alkyle avec 1 à 20 atomes de carbone,
Z² est le groupe 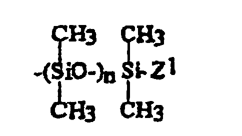 R¹, R², R³ et R⁴
sont identiques ou différents et signifient des radicaux alkyles avec 1 à 4 atomes de carbone ou des radicaux benzyles, ou dans lesquels les radicaux R¹ et R³ ou R² et R⁴ respectivement peuvent être des composants d'un radical alkylène à effet de pontage,
R⁶ signifie un H ou un radical alkyle avec 1 à 20 atomes de carbone, qui peut être substitué par de l'oxygène
E signifie la structure -B-(OCH₂CH₂)ᵥ(OCH₂CH(CH₃))_{w}-O-B- avec
B signifie un alkylène linéaire ou ramifié en C₂ à C₆,
v signifie 0 à 200
w signifie 0 à 200
v + w correspond à ≥1
A' signifie un anion inorganique ou organique
n signifie 5 à 200
m signifie un nombre entier ≥1 et
s signifie un nombre entier ≥1.

5. Polymères de polysiloxane selon l'une quelconque des revendications 1 à 4, **caractérisés en ce que** n est = 5 à 82.

6. Polymères de polysiloxane selon l'une quelconque des revendications 1 à 5, **caractérisés en ce que** n est = 5 à 20

7. Polymères de polysiloxane selon l'une quelconque des revendications 1 à 7, **caractérisés en ce que** X représente un radical parmi le groupe 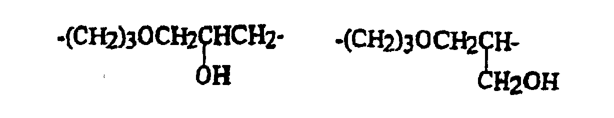 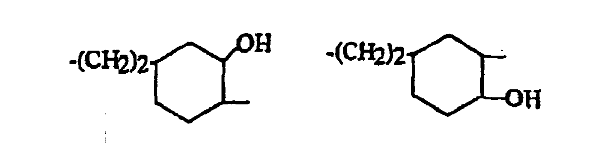 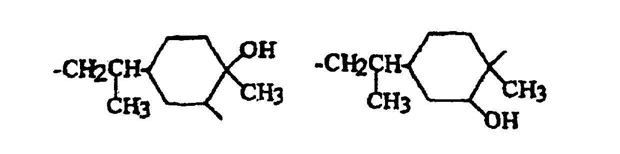 Y est un radical -(CH₂)ₒ-, avec o de 2 à 6, les groupes R¹, R², R³, R⁴ sont des radicaux méthyles, et R⁶ est un hydrogène, un -CH₂-CH₂-OH et un -CH₃.

8. Polymères de polysiloxane selon l'une quelconque des revendications 1 à 7, **caractérisés en ce que** l'élément structurel B est représenté par des unités -CH₂-CH₂- et -CH₂₋CH(CH₃)-.

9. Polymères de polysiloxane selon l'une quelconque des revendications 1 à 8, **caractérisés en ce que** Y signifie -(CH₂)ₒ-, avec o de 2 à 6.

10. Polymères de polysiloxane selon l'une quelconque des revendications 1 à 9, **caractérisés en ce que** R¹, R², R³, R⁴ sont des méthyles.

11. Polymères de polysiloxane selon l'une quelconque des revendications 1 à 10, **caractérisés en ce que** R⁶ est un hydrogène, un -CH₂CH₂OH et un -CH₃.

12. Polymères de polysiloxane selon l'une quelconque des revendications 1 à 11, **caractérisés en ce que** B représente des unités -CH₂CH₂- et -CH₂CH(CH₃)-.

13. Polymères de polysiloxane selon l'une quelconque des revendications 1 à 12, **caractérisés en ce que** v signifie 0 à 100.

14. Polymères de polysiloxane selon l'une quelconque des revendications 4 à 13, **caractérisés en ce que** m/(m+s) x 100 = 0,1 à 99,9 %.

15. Polymères de polysiloxane selon l'une quelconque des revendications 1 à 14, **caractérisés en ce que** w signifie 0 à 100.

16. Polymères de polysiloxane selon l'une quelconque des revendications 1 à 15, **caractérisés en ce que** l'on utilise comme anions A⁻ des radicaux inorganiques physiologiquement acceptables du groupe consistant en le chlorure, le bromure, l'hydrogénosulfate, le sulfate, ou des radicaux organiques du groupe consistant en l'acétate, le propionate, l'octanoate, le décanoate, le dodécanoate, le tétradécanoate, l'hexadécanoate, l'octadécanoate, l'oléate.

17. Polymères de polysiloxane selon l'une quelconque des revendications 1 à 16, **caractérisés en ce que** les composés se présentent sous forme protonée comme sels d'amines.

18. Procédé de fabrication de polymères de polysiloxane selon l'une quelconque des revendications 1 à 17, **caractérisé en ce que** l'on fait réagir des polysiloxanes à terminaison bisépoxyde de la formule 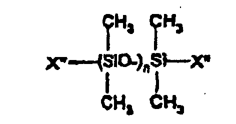 dans laquelle X" est un radical hydrocarbure divalent avec au moins 4 atomes de carbone, qui comporte un groupe époxyde et qui peut être interrompu par un atome d'oxygène, avec des bis amines des formules 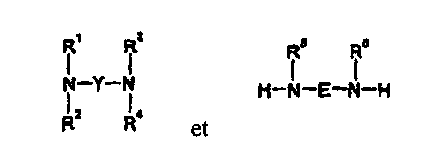 dans un ordre approprié, le cas échéant moyennant l'addition d'une monoamine de la formule 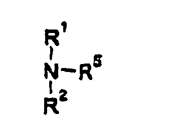 dans lesquelles les substituants sont définis comme ci-dessus.

19. Polymères de polysiloxane selon l'une quelconque des revendications 1 à 17 pour une utilisation comme plastifiants hydrophiles sur la base de siloxanes contenant des groupes ammoniums quaternaires.

20. Utilisation des polymères de polysiloxane selon l'une quelconque des revendications 1 à 17 et 19, comme plastifiants hydrophiles résistant au lessivage.

21. Utilisation des polymères de polysiloxane selon la revendication 20 comme plastifiants hydrophiles résistant au lessivage pour le premier apprêt de textiles.

22. Utilisation des composés de polysiloxane selon l'une quelconque des revendications 1 à 17, dans des formulations cosmétiques pour les soins de la peau et des cheveux, dans des vernis pour le traitement et l'apprêt de surfaces dures, dans des formulations pour le séchage d'automobiles et d'autres surfaces dures après des lavages mécaniques, pour l'apprêt de textiles et de fibres textiles, comme plastifiants séparés après le lavage de textiles avec des formulations de détergents non ionogènes ou anioniques/non ionogènes, comme plastifiants dans des formulations pour le lavage des textiles basées sur des tensioactifs non ionogènes ou anioniques/non ionogènes.
